(19) 

**Europäisches Patentamt**
**European Patent Office**
**Office européen des brevets**

(11) **EP 4 243 694 B1**

(12) **EUROPEAN PATENT SPECIFICATION**

(45) Date of publication and mention
of the grant of the patent:
**04.09.2024 Bulletin 2024/36**

(21) Application number: **21830881.5**

(22) Date of filing: **03.12.2021**

(51) International Patent Classification (IPC):
**A61B 5/352** (2021.01)    **A61B 5/024** (2006.01)
**A61B 5/00** (2006.01)

(52) Cooperative Patent Classification (CPC):
**A61B 5/352; A61B 5/02438; A61B 5/7221**

(86) International application number:
**PCT/US2021/061735**

(87) International publication number:
**WO 2022/120125 (09.06.2022 Gazette 2022/23)**

(54) **TIME DOMAIN PROCESSING OF PERIODIC PHYSIOLOGICAL SIGNALS**

ZEITBEREICHSVERARBEITUNG VON PERIODISCHEN PHYSIOLOGISCHEN SIGNALEN

TRAITEMENT DANS LE DOMAINE TEMPOREL DE SIGNAUX PHYSIOLOGIQUES PÉRIODIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.12.2020 US 202063121636 P**

(43) Date of publication of application:
**20.09.2023 Bulletin 2023/38**

(73) Proprietor: **Whoop, Inc.**
**Boston, MA 02215 (US)**

(72) Inventors:
• **ARGYROPOULOS, Paraskevas**
**Boston, MA 02215 (US)**
• **GHANNAD-REZAIE, Mostafa**
**Boston, MA 02215 (US)**

(74) Representative: **Black, Diego**
**Withers & Rogers LLP**
**2 London Bridge**
**London SE1 9RA (GB)**

(56) References cited:
**US-A1- 2016 106 320**

• **RAJU DANDU SRIRAM ET AL: "An automated
method for detecting systolic peaks from arterial
blood pressure signals", PROCEEDINGS OF THE
2014 IEEE STUDENTS' TECHNOLOGY
SYMPOSIUM, IEEE, 28 February 2014
(2014-02-28), pages 41 - 46, XP032778476, DOI:
10.1109/TECHSYM.2014.6807911**
• **BHATTACHARJEE TANUKA ET AL: "Robust
Beat-to-Beat Interval from Wearable PPG using
RLS and SSA", 2019 41ST ANNUAL
INTERNATIONAL CONFERENCE OF THE IEEE
ENGINEERING IN MEDICINE AND BIOLOGY
SOCIETY (EMBC), IEEE, 23 July 2019
(2019-07-23), pages 4946 - 4952, XP033625048,
DOI: 10.1109/EMBC.2019.8857140**
• **PELTOKANGAS M ET AL: "Night-Time EKG and
HRV Monitoring With Bed Sheet Integrated
Textile Electrodes", IEEE TRANSACTIONS ON
INFORMATION TECHNOLOGY IN BIOMEDICINE,
IEEE SERVICE CENTER, LOS ALAMITOS, CA, US,
vol. 16, no. 5, 1 September 2012 (2012-09-01),
pages 935 - 942, XP011490956, ISSN: 1089-7771,
DOI: 10.1109/TITB.2012.2208982**
• **"A Method and Apparatus for Multi-Stage Data
Quality Indexing of Cardiovascular Signals from
Wearable Photoplethysmogram Sensors ED -
Darl Kuhn", IP.COM, IP.COM INC., WEST
HENRIETTA, NY, US, 13 May 2020 (2020-05-13),
XP013186598, ISSN: 1533-0001**

EP 4 243 694 B1

- A. GRABOVSKIS ET AL: "Photoplethysmography system for blood pulsation detection in unloaded artery conditions", SPIE PROCEEDINGS, vol. 8427, 26 April 2012 (2012-04-26), US, pages 84270L, XP055607104, ISBN: 978-1-5106-3673-6, DOI: 10.1117/12.922649

## Description

CROSS-REFERENCE TO RELATED APPLICATIONS

This application claims priority to U.S. Provisional Application No. 63/121,636 filed on December 4, 2020.

BACKGROUND

Time domain algorithms may be used to provide reliable peak-to-peak estimates in physiological data. However, time domain processing may fail to accurately identify pulses in physiological data that contain unusual periodic pulse shapes resulting from, e.g., a medical pathology, extremes in a fitness level or activity level, an unusual physiological condition, and so forth. There is a need for time domain processing techniques capable of accurately detecting physiological pulse intervals over a wider range of users and activities.

RAJU DANDU SRIRAM ET AL, "An automated method for detecting systolic peaks from arterial blood pressure signals", PROCEEDINGS OF THE 2014 IEEE STUDENTS' TECHNOLOGY SYMPOSIUM, IEEE, (20140228), discloses an automatic method for determining time-location of systolic peak in arterial blood pressure (ABP) signals. The method consists of four major steps: Gaussian derivative filtering, nonlinear peak amplification, Gaussian derivative based peak finding scheme, and peak position adjustment procedure.

A. GRABOVSKIS ET AL, "Photoplethysmography system for blood pulsation detection in unloaded artery conditions", SPIE PROCEEDINGS, US, (20120426), vol. 8427, discloses a study to develop and evaluate a system for software-assisted PPG signal acquisition from the unloaded artery. Novel PPG waveform derived Optimal Pressure Parameter (OPP) is proposed as the reliable indicator of unloaded artery condition.

SUMMARY

A periodic physiological signal such as pulse data can be analyzed in the time domain to identify peaks and other features of interest, and to evaluate how well the signal corresponds to an expected shape. For example, state machines may be used to sequentially analyze samples of time domain data and perform peak detection, signal quality analysis, and so forth. This time domain analysis model permits adaptations to known variations in typical signals, and advantageously enables accurate physiological inferences over a greater range of user-specific contexts including different activity types, fitness levels, and medical conditions.

In an aspect, a computer program product disclosed herein includes a computer program product for operating a wearable, continuous physiological monitoring system, the computer program product comprising computer executable code embodied in a non-transitory computer readable medium that, when executing on the wearable, continuous physiological monitoring system, performs the steps of: storing a window of time domain data for a physiological signal, the window including a series of samples of the physiological signal; detecting a first peak and a second peak in the physiological signal using a time domain process on the series of samples, wherein the time domain process includes a first state machine configured to sequentially process the series of samples and identify the first peak and the second peak in the series of samples; evaluating a quality of the time domain data in the window indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine; locally refining a peak position of each of the first peak and the second peak, wherein locally refining the peak position includes interpolating a location of a maximum for a number of sequential samples within the time domain data having similar magnitudes; and when the quality exceeds a predetermined threshold, conditionally calculating a physiological interval for the window of time domain data wherein the physiological interval is based on a time between the first peak and the second peak and storing a value based on the quality as a quality flag for the window of time domain data.

Implementations may include one or more of the following features. The physiological signal may be a heart rate signal, and evaluating the quality of the time domain data may further include conditionally labelling the quality of the time domain data as low-RR-quality when the difference in amplitudes of the first local maximum and the second local maximum are within a predetermined threshold. The first state machine may be further configured to perform the steps of: identifying a first pulse candidate, wherein the first pulse candidate is a heart rate signal; conditionally labelling the first pulse candidate as the first peak when the first pulse candidate is determined to be an R-pulse; identifying a second pulse candidate, wherein the second pulse candidate is a heart rate signal; and conditionally labelling the second pulse candidate as the second peak when the second pulse candidate is determined to be an R-pulse.

In an aspect, a method disclosed herein includes storing a window of time domain data for a physiological signal, the window including a series of samples of the physiological signal; detecting a first peak and a second peak in the physiological signal using a time domain process on the series of samples; wherein the time domain process includes a first state machine (800) configured to sequentially process the series of samples and identify the first peak and the second peak in the series of samples; evaluating a quality of the time domain data in the window indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine (900); locally refining a peak position of each of the first peak and the second peak, wherein locally refining the peak position includes interpolating a location of a maximum for a number of sequential samples within the time domain data having similar magnitudes; and when the quality exceeds a predetermined threshold, conditionally calculating a physiological interval for the window of time domain data wherein the physiological interval is based on a time between the first peak and the second peak and storing a value based on the quality as a quality flag for the window of time domain data..

Implementations may include one or more of the following features. The method may further include evaluating a quality of the time domain data in the window indicative of how well a time domain shape associated with a peak shape matches an expected peak shape for the physiological signal; conditionally calculating the physiological interval when the quality exceeds a predetermined threshold; and storing a value based on the quality as a quality flag for the window. Evaluating a quality of the time domain data may include identifying the peak shape by identifying a local minimum located between a first local maximum and a second local maximum with a state machine. The physiological signal may be a heart rate signal, and evaluating the quality of the time domain data may further include conditionally labelling the quality of the time domain data as low-RR-quality when the difference in amplitudes of the first local maximum and the second local maximum are within a predetermined threshold. Locally refining the peak position may include interpolating a location of a maximum for a number of sequential samples within the time domain data having similar magnitudes. Locally refining the peak position may include estimating a value of a maximum at a location for the maximum between two of the sequential samples within the time domain data. The time domain process may include a state machine configured to sequentially process the series of samples and identify the first peak and the second peak in the series of samples. The state machine may be further configured to perform the steps of: identifying a first pulse candidate, wherein the first pulse candidate is a heart rate signal; conditionally labelling the first pulse candidate as the first peak when the first pulse candidate is determined to be an R-pulse; identifying a second pulse candidate, wherein the second pulse candidate is a heart rate signal; and conditionally labelling the second pulse candidate as the second peak when the second pulse candidate is determined to be an R-pulse. The state machine may be further configured to compare second-order statistics of the first peak and the second peak.

In an aspect, a system disclosed herein includes: a wearable housing; one or more sensors in the wearable housing for capturing physiological data; and a processor in the wearable housing, the processor configured by computer executable code to perform the steps of: storing a window of time domain data for a physiological signal, the window including a series of samples of the physiological signal; detecting a first peak and a second peak in the physiological signal using a time domain process on the series of samples, wherein the time domain process includes a first state machine (800) configured to sequentially process the series of samples and identify the first peak and the second peak in the series of samples; evaluating a quality of the time domain data in the window indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine (900); locally refining a peak position of each of the first peak and the second peak, wherein locally refining the peak position includes interpolating a location of a maximum for a number of sequential samples within the time domain data having similar magnitudes; and when the quality exceeds a predetermined threshold, conditionally calculating a physiological interval for the window of time domain data wherein the physiological interval is based on a time between the first peak and the second peak and storing a value based on the quality as a quality flag for the window of time domain data..

Implementations may include one or more of the following features. The processor may be further configured to perform the steps of: evaluating a quality of the time domain data in the window indicative of how well a time domain shape associated with a peak shape matches an expected peak shape for the physiological signal; conditionally calculating the physiological interval when the quality exceeds a predetermined threshold; and storing a value for a quality flag based on the quality. Evaluating a quality of the time domain data may include identifying the peak shape by identifying a local minimum located between a first local maximum and a second local maximum with a state machine. Locally refining the peak position may include interpolating a location of a maximum for a number of sequential samples within the time domain data having similar magnitudes. Locally refining the peak position may include estimating a value of a maximum at a location for the maximum between two sequential samples of the time domain data. The time domain process may include a state machine configured to sequentially process the series of samples and identify the first peak and the second peak in the series of samples. The state machine may be further configured to perform the steps of: identifying a first pulse candidate, wherein the first pulse candidate is a heart rate signal; conditionally labelling the first pulse candidate as the first peak when the first pulse candidate is determined to be an R-pulse; identifying a second pulse candidate, wherein the second pulse candidate is a heart rate signal; and conditionally labelling the second pulse candidate as the second peak when the second pulse candidate is determined to be an R-pulse. The state machine may be further configured to compare second-order statistics of the first peak and the second peak.

BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.

Fig. 1 illustrates front and back perspective views of a wearable system configured as a bracelet including one or more straps.
Fig. 2 shows a block diagram illustrating components of a wearable physiological measurement system configured to provide continuous collection and monitoring of physiological data.
Fig. 3 is a flow chart illustrating a signal processing algorithm for generating a sequence of heart rates for every detected heartbeat that may be embodied in computer-executable instructions stored on one or more non-transitory computer-readable media.
Fig. 4 is a flow chart illustrating a method of determining an intensity score.
Fig. 5 is a flow chart illustrating a method by which a user may use intensity and recovery scores.
Fig. 6 is a flow chart illustrating a method for detecting heart rate variability in sleep states.
Fig. 7 is a bottom view of a wearable, continuous physiological monitoring device.
Fig. 8 shows a state machine diagram for processing a buffer of optical data.
Fig. 9 shows a state machine diagram for identifying accuracy of a pulse.
Fig. 10 is a graph including a time series of a time domain buffer of optical data.
Fig. 11 is a graph including a time series of a time domain buffer of optical data.
Fig. 12 is a flow chart illustrating a method for time domain processing of periodic physiological signals.
Fig. 13 shows a first graph including a time domain buffer of optical data and a second graph showing state machine debugging information.
Fig. 14 shows a first graph including a time domain buffer of optical data and a second graph showing state machine debugging information.
Fig. 15 shows a first graph including a time domain buffer of optical data and a second graph showing state machine debugging information.
Fig. 16 shows a first graph including a time domain buffer of optical data and a second graph showing state machine debugging information.

DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the embodiments. No language in the specification should be construed as indicating any unclaimed element as essential to the practice of the embodiments.

In the following description, it is understood that terms such as "first," "second," "above," "below," and the like, are words of convenience and are not to be construed as limiting terms.

Exemplary embodiments provide physiological measurement systems, devices and methods for continuous health and fitness monitoring, and provide improvements to overcome the drawbacks of conventional heart rate monitors. One aspect of the present disclosure is directed to providing a lightweight wearable system with a strap that collects various physiological data or signals from a wearer. The strap may be used to position the system on an appendage or extremity of a user, for example, wrist, ankle, and the like. Exemplary systems are wearable and enable real-time and continuous monitoring of heart rate without the need for a chest strap or other bulky equipment which could otherwise cause discomfort and prevent continuous wearing and use. The system may determine the user's heart rate without the use of electrocardiography and without the need for a chest strap. Exemplary systems can thereby be used in not only assessing general well-being but also in continuous monitoring of fitness. Exemplary systems also enable monitoring of one or more physiological parameters in addition to heart rate including, but not limited to, body temperature, heart rate variability, motion, sleep, stress, fitness level, recovery level, effect of a workout routine on health and fitness, caloric expenditure, and the like.

A health or fitness monitor that includes bulky components may hinder continuous wear. Existing fitness monitors often include the functionality of a watch, thereby making the health or fitness monitor quite bulky and inconvenient for continuous wear. Accordingly, one aspect is directed to providing a wearable health or fitness system that does not include bulky components, thereby making the bracelet slimmer, unobtrusive and appropriate for continuous wear. The ability to continuously wear the bracelet further allows continuous collection of physiological data, as well as continuous and more reliable health or fitness monitoring. For example, embodiments of the bracelet disclosed herein allow users to monitor data at all times, not just during a fitness session. In some embodiments, the wearable system may or may not include a display screen for displaying heart rate and other information. In other embodiments, the wearable system may include one or more light emitting diodes (LEDs) to provide feedback to a user and display heart rate selectively. In some embodiments, the wearable system may include a removable or releasable modular head that may provide additional features and may display additional information. Such a modular head can be releasably installed on the wearable system when additional information display is desired, and removed to improve the comfort and appearance of the wearable system. In other embodiments, the head may be integrally formed in the wearable system.

Exemplary embodiments also include computer-executable instructions that, when executed, enable automatic interpretation of one or more physiological parameters to assess the cardiovascular intensity experienced by a user (embodied in an intensity score or indicator) and the user's recovery after physical exertion or daily stress given sleep and other forms of rest (embodied in a recovery score). These indicators or scores may be stored and displayed in a meaningful format to assist a user in managing his health and exercise regimen.

In an exemplary technique of data transmission, data collected by a wearable system may be transmitted directly to a cloud-based data storage, from which data may be downloaded for display and analysis on a website. In another exemplary technique of data transmission, data collected by a wearable system may be transmitted via a mobile communication device application to a cloud-based data storage, from which data may be downloaded for display and analysis on a website.

The term "user" as used herein, refers to any type of animal, human or non-human, whose physiological information may be monitored using an exemplary wearable physiological monitoring system. The term "body," as used herein, refers to the body of a user.

The term "continuous," as used herein in connection with heart rate data collection, refers to collection of heart rate data at a sufficient frequency to enable detection of every heartbeat and also refers to collection of heart rate data continuously throughout the day and night.

The term "computer-readable medium," as used herein, refers to a non-transitory storage hardware, non-transitory storage device or non-transitory computer system memory that may be accessed by a controller, a microcontroller, a computational system or a module of a computational system to encode thereon computer-executable instructions or software programs. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), computer system memory or random access memory (such as, DRAM, SRAM, EDO RAM) and the like.

Exemplary embodiments provide wearable physiological measurements systems that are configured to provide continuous measurement of heart rate. Exemplary systems are configured to be continuously wearable on an appendage, for example, wrist or ankle, and do not rely on electrocardiography or chest straps in detection of heart rate. The exemplary system includes one or more light emitters for emitting light at one or more desired frequencies toward the user's skin, and one or more light detectors for received light reflected from the user's skin. The light detectors may include a photo-resistor, a photo-transistor, a photo-diode, and the like. As light from the light emitters (for example, green light) pierces through the skin of the user, the blood's natural absorbance or transmittance for the light provides fluctuations in the photo-resistor readouts. These waves have the same frequency as the user's pulse since increased absorbance or transmittance occurs only when the blood flow has increased after a heartbeat. The system includes a processing module implemented in software, hardware or a combination thereof for processing the optical data received at the light detectors and continuously determining the heart rate based on the optical data. The optical data may be combined with data from one or more motion sensors, e.g., accelerometers and/or gyroscopes, to minimize or eliminate noise in the heart rate signal caused by motion or other artifacts (or with other optical data of another wavelength).

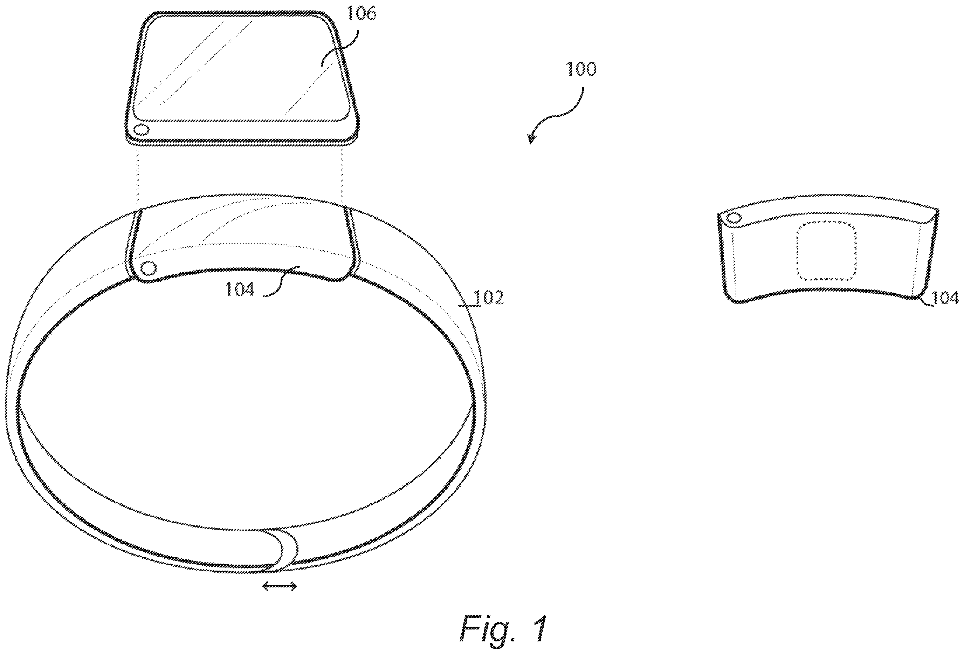

Fig. 1 illustrates front and back perspective views of one embodiment of a wearable system configured as a bracelet 100 including one or more straps 102. The bracelet is sleek and lightweight, thereby making it appropriate for continuous wear. The bracelet may or may not include a display screen, e.g., a screen 106 such as a light emitting diode (LED) display for displaying any desired data (e.g., instantaneous heart rate).

As shown in Fig. 1, the wearable system may include components configured to provide various functions such as data collection and streaming functions of the bracelet. In some embodiments, the wearable system may include a button underneath the wearable system. In some embodiments, the button may be configured such that, when the wearable system is properly tightened to one's wrist, the button may press down and activate the bracelet to begin storing information. In other embodiments, the button may be disposed and configured such that it may be pressed manually at the discretion of a user to begin storing information or otherwise to mark the start or end of an activity period such as sleep. In some embodiments, the button may be held to initiate a time stamp and held again to end a time stamp, which may be transmitted, directly or through a mobile communication device application, to a website as a time stamp.

The wearable system may include a heart rate monitor. In one example, the heart rate may be detected from the radial artery. Thus, the wearable system may include a pulse sensor. In one embodiment, the wearable system may be configured such that, when a user wears it around their wrist and tightens it, the sensor portion of the wearable system is secured over the user's radial artery or other blood vessel. Secure connection and placement of the pulse sensor over the radial artery or other blood vessel may allow measurement of heart rate and pulse. It will be understood that this configuration is provided by way of example only, and that other sensors, sensor positions, and monitoring techniques may also or instead be employed without departing from the scope of this disclosure.

In some embodiments, the pulse or heart rate may be taken using an optical sensor coupled with one or more light emitting diodes (LEDs), all directly in contact with the user's wrist. The LEDs may be provided in a suitable position from which light can be emitted into the user's skin. In one example, the LEDs may be mounted on a side or top surface of a circuit board in the system to prevent heat buildup on the LEDs and to prevent burns on the skin. The circuit board may be designed with the intent of dissipating heat, e.g., by including thick conductive layers, exposed copper, heatsink, or similar. In one aspect, the pulse repetition frequency is such that the amount of power thermally dissipated by the LED is negligible. Cleverly designed elastic wrist straps can ensure that the sensors are always in contact with the skin and that there is a minimal amount of outside light seeping into the sensors. In addition to the elastic wrist strap, the design of the strap may allow for continuous micro adjustments (no preset sizes) in order to achieve an optimal fit, and a floating sensor module. The sensor module may be free to move with the natural movements caused by flexion and extension of the wrist.

In some embodiments, the wearable system may be configured to record other physiological parameters including, but not limited to, skin temperature (using a thermometer), galvanic skin response (using a galvanic skin response sensor), motion (using one or more multi-axes accelerometers and/or gyroscope), and the like, and environmental or contextual parameters, e.g., ambient temperature, humidity, time of day, and the like. In an implementation, sensors may be used to provide at least one of continuous motion detection, environmental temperature sensing, electrodermal activity (EDA) sensing, galvanic skin response (GSR) sensing, and the like. In this manner, an implementation can identify the cause of a detected physiological event. Reflectance PhotoPlethysmoGraphy (RPPG) may be used for the detection of cardiac activity, which may provide for non-intrusive data collection, usability in wet, dusty and otherwise harsh environments, and low power requirements. For example, as explained herein, using the physiological readouts of the device and the analytics described herein, an "Intensity Score" (e.g., 0-21) (e.g., that measures a user's recent exertion), a "Recovery Score" (e.g., 0-100%), and "Sleep Score" (e.g., 0-100) may together measure readiness for physical and psychological exertion.

In some embodiments, the wearable system may further be configured such that a button underneath the system may be pressed against the user's wrist, thus triggering the system to begin one or more of collecting data, calculating metrics and communicating the information to a network. In some embodiments, the sensor used for, e.g., measuring heart rate or GSR or any combination of these, may be used to indicate whether the user is wearing the wearable system or not. In some embodiments, power to the one or more LEDs may be cut off as soon as this situation is detected and reset once the user has put the wearable system back on their wrist.

The wearable system may include one or more sources of battery life, e.g., two or more batteries. In some embodiments, the wearable system may have a battery that can slip in and out of the head of the wearable system and can be recharged using an included accessory. Additionally, the wearable system may have a built-in battery that is less powerful. When the more powerful battery is being charged, the user does not need to remove the wearable system and can still record data (during sleep, for example).

In exemplary embodiments, the wearable system may be enabled to automatically detect when the user is asleep, awake but at rest, and exercising based on physiological data collected by the system.

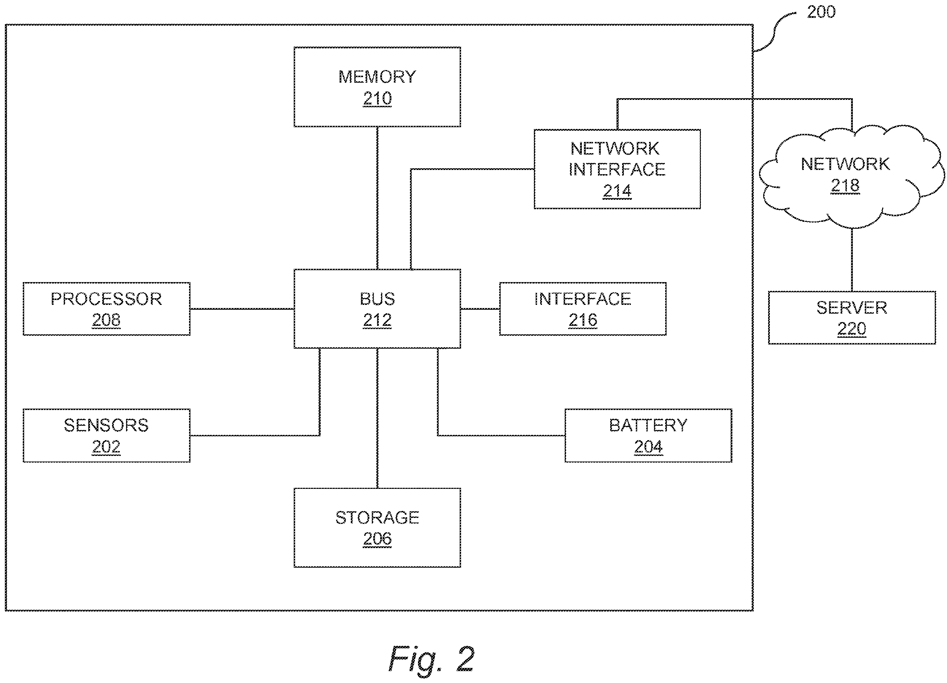

Fig. 2 shows a block diagram illustrating exemplary components of a wearable physiological measurement system 200 configured to provide continuous collection and monitoring of physiological data. The wearable system 200 may include one or more sensors 202. As discussed above, the sensors 202 may include a heart rate monitor. In some embodiments, the wearable system 200 may further include one or more of sensors for detecting calorie burn, distance and activity. Calorie burn may be based on a user's heart rate, and a calorie burn measurement may be improved if a user chooses to provide his or her weight and/or other physical parameters. In some embodiments, manual entering of data may not be required to derive calorie burn; however, data entry may be used to improve the accuracy of the results. In some embodiments, if a user has forgotten to enter a new weight, the user may enter it for past weeks and the calorie burn may be updated accordingly.

The sensors 202 may include one or more sensors for activity measurement. In some embodiments, the system may include one or more multi-axes accelerometers and/or gyroscope to provide a measurement of activity. In some embodiments, the accelerometer may further be used to filter a signal from the optical sensor for measuring heart rate and to provide a more accurate measurement of the heart rate. In some embodiments, the wearable system may include a multi-axis accelerometer to measure motion and calculate distance, whether it be in real terms as steps or miles or as a converted number. Activity sensors may be used, for example, to classify or categorize activity, such as walking, running, performing another sport, standing, sitting or lying down. In some embodiments, one or more of collected physiological data may be aggregated to generate an aggregate activity level. For example, heart rate, calorie burn, and distance may be used to derive an aggregate activity level. The aggregate level may be compared with or evaluated relative to previous recordings of the user's aggregate activity level, as well as the aggregate activity levels of other users.

The sensors 202 may include a thermometer for monitoring the user's body or skin temperature. In one embodiment, the sensors may be used to recognize sleep based on a temperature drop, GSR data, lack of activity according to data collected by the accelerometer, and reduced heart rate as measured by the heart rate monitor. The body temperature, in conjunction with heart rate monitoring and motion, may be used to interpret whether a user is sleeping or just resting, as body temperature drops significantly when an individual is about to fall asleep, and how well an individual is sleeping as motion indicates a lower quality of sleep. The body temperature may also be used to determine whether the user is exercising and to categorize and/or analyze activities.

The system 200 may include one or more batteries 204. According to one embodiment, the one or more batteries may be configured to allow continuous wear and usage of the wearable system. In one embodiment, the wearable system may include two or more batteries. The system may include a removable battery that may be recharged using a charger. In one example, the removable battery may be configured to slip in and out of a head portion of the system, attach onto the bracelet, or the like. In one example, the removable battery may be able to power the system for around a week. Additionally, the system may include a built-in battery. The built-in battery may be recharged by the removable battery. The built-in battery may be configured to power the bracelet for around a day on its own. When the more removable battery is being charged, the user does not need to remove the system and may continue collecting data using the built-in battery. In other embodiments, the two batteries may both be removable and rechargeable.

In some embodiments, the system 200 may include a battery that is a wireless rechargeable battery. For example, the battery may be recharged by placing the system or the battery on a rechargeable mat. In other example, the battery may be a long range wireless rechargeable battery. In other embodiments, the battery may be a rechargeable via motion. In yet other embodiments, the battery may be rechargeable using a solar energy source.

The wearable system 200 may include one or more non-transitory computer-readable media 206 for storing raw data detected by the sensors of the system and processed data calculated by a processing module of the system.

The system 200 may include a processor 208, a memory 210, a bus 212, a network interface 214, and an interface 216. The network interface 214 is configured to wirelessly communicate data to an external network 218. The network 218 may include any communication network through which computer systems may exchange data. For example, the network 218 may include, but is not limited to, the Internet, an intranet, a LAN (Local Area Network), a WAN (Wide Area Network), a MAN (Metropolitan Area Network), a wireless network, an optical network, and the like. To exchange data via the network 218, the system 200 and the network 218 may use various methods, protocols and standards including, but not limited to, token ring, Ethernet, wireless Ethernet, Bluetooth, TCP/IP, UDP, HTTP, FTP, SNMP, SMS, MMS, SS7, JSON, XML, REST, SOAP, CORBA, IIOP, RMI, DCOM and Web Services. To ensure data transfer is secure, the system 200 may transmit data via the network using a variety of security measures including, but not limited to, TSL, SSL and VPN.

Some embodiments of the wearable system may be configured to stream information wirelessly to a social network. In some embodiments, data streamed from a user's wearable system to an external network 218 may be accessed by the user via a website. The network interface may be configured such that data collected by the system may be streamed wirelessly. In some embodiments, data may be transmitted automatically, without the need to manually press any buttons. In some embodiments, the system may include a cellular chip built into the system. In one example, the network interface may be configured to stream data using Bluetooth technology. In another example, the network interface may be configured to stream data using a cellular data service, such as via a 3G or 4G cellular network.

The system 200 may be coupled to one or more servers 220 via a communication network 218.

In some embodiments, a physiological measurement system may be configured in a modular design to enable continuous operation of the system in monitoring physiological information of a user wearing the system. The module design may include a strap and a separate modular head portion or housing that is removably couplable to the strap.

In the non-limiting illustrative module design, the strap 102 of a physiological measurement system may be provided with a set of components that enables continuous monitoring of at least a heart rate of the user so that it is independent and fully self-sufficient in continuously monitoring the heart rate without requiring the modular head portion 104. In one embodiment, the strap may include a plurality of light emitters for emitting light toward the user's skin, a plurality of light detectors for receiving light reflected from the user's skin, an electronic circuit board comprising a plurality of electronic components configured for analyzing data corresponding to the reflected light to automatically and continually determine a heart rate of the user, and a first set of one or more batteries for supplying electrical power to the light emitters, the light detectors and the electronic circuit board. In some embodiments, the strap may also detect one or more other physiological characteristics of the user including, but not limited to, temperature, galvanic skin response, and the like.

Certain exemplary systems may be configured to be coupled to any desired part of a user's body so that the system may be moved from one portion of the body (e.g., wrist) to another portion of the body (e.g., ankle) without affecting its function and operation. In one embodiment, the identity of the portion of the user's body to which the wearable system is attached may be determined based on one or more parameters including, but not limited to, absorbance level of light as returned from the user's skin, reflectance level of light as returned from the user's skin, motion sensor data (e.g., accelerometer and/or gyroscope), altitude of the wearable system, and the like.

In some embodiments, the processing module may be configured to determine that the wearable system is taken off from the user's body. In one example, the processing module may determine that the wearable system has been taken off if data from the galvanic skin response sensor indicates data atypical of a user's skin. If the wearable system is determined to be taken off from the user's body, the processing module may be configured to deactivate the light emitters and the light detectors and cease monitoring of the heart rate of the user to conserve power.

Exemplary systems may include a processing module configured to filter the raw photoplethysmography data received from the light detectors to minimize contributions due to motion, and subsequently process the filtered data to detect peaks in the data that correspond with heart beats of a user. The overall algorithm for detecting heart beats may take as input the analog signals from optical sensors (mV) and accelerometer and output an implied beats per minute (heart rate) of the signal accurate within a few beats per minute of that determined by an electrocardiography machine even during motion.

In one aspect, using multiple LEDs with different wavelengths reacting to movement in different ways may allow for signal recovery with standard signal processing techniques. The availability of accelerometer information may also be used to compensate for coarse movement signal corruption. In order to increase the range of movements that the algorithm can successfully filter out, an aspect may use techniques that augment the algorithm already in place. For example, filtering violent movements of the arm during very short periods of time, such as boxing as exercising, may be utilized by the system. By selective sampling and interpolating over these impulses, an aspect may account for more extreme cases of motion. Additionally, an investigation into different LED wavelengths, intensities, and configurations can allow the systems described herein to extract a signal across a wide spectrum of skin types and wrist sizes. In other words, motion filtering algorithms and signal processing techniques may assist in mitigating the risk caused by movement.

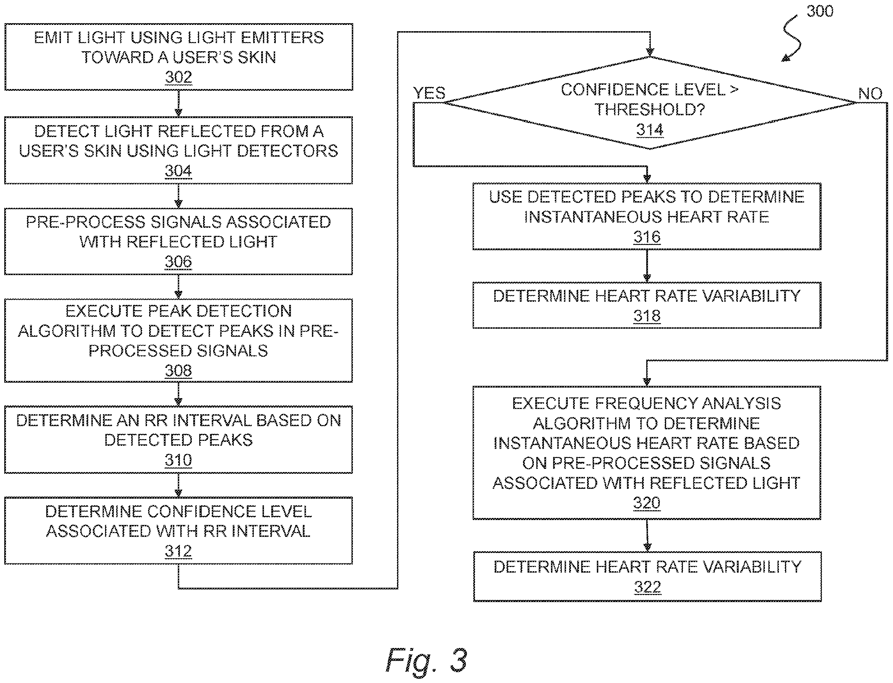

Fig. 3 is a flow chart illustrating an exemplary signal processing algorithm for generating a sequence of heart rates for every detected heartbeat that is embodied in computer-executable instructions stored on one or more non-transitory computer-readable media. The method 300 may be performed, e.g., by a processor or other processing circuitry or processing module of a wearable system, and/or a remote processing resource associated with the wearable system.

As shown in step 302, the method 300 may include emitting light toward a user's skin with light emitters of a wearable physiological measurement system. As shown in step 304, the method 300 may include detecting light reflected from the user's skin at the light detectors in the system. As shown in step 306, the method 300 may include pre-processing signals or data associated with the reflected light using any suitable technique to facilitate detection of heart beats. As shown in step 308, the method 300 may include executing one or more computer-executable instructions associated with a peak detection algorithm to process data corresponding to the reflected light to detect a plurality of peaks associated with a plurality of beats of the user's heart. As shown in step 310, the method 300 may include determining an RR interval based on the plurality of peaks detected by the peak detection algorithm.

As shown in step 312, the method 300 may include determining a confidence level associated with the RR interval. Based on the confidence level associated with the RR interval estimate, the processing module may select either the peak detection algorithm or a frequency analysis algorithm to process data corresponding to the reflected light to determine the sequence of instantaneous heart rates of the user. The frequency analysis algorithm may process the data corresponding to the reflected light based on the motion of the user detected using, for example, an accelerometer. The processing module may select the peak detection algorithm or the frequency analysis algorithm regardless of a motion status of the user. The processing module may use a confidence in the estimate in deciding whether to switch to frequency-based methods as certain frequency-based approaches are unable to obtain accurate RR intervals for heart rate variability analysis. Therefore, an implementation can maintain the ability to obtain the RR intervals for as long as possible, even in the case of motion, thereby maximizing the information that can be extracted.

For example, as shown in step 314, the method 300 may include determining whether the confidence level associated with the RR interval is above (or equal to or above) a threshold. In certain embodiments, the threshold may be predefined, for example, about 50%-90% in some embodiments and about 80% in one non-limiting embodiment. In other embodiments, the threshold may be adaptive, i.e., the threshold may be dynamically and automatically determined based on previous confidence levels. For example, if one or more previous confidence levels were high (i.e., above a certain level), the system may determine that a present confidence level that is relatively low compared to the previous levels is indicative of a less reliable signal. In this case, the threshold may be dynamically adjusted to be higher so that a frequency-based analysis method may be selected to process the less reliable signal.

If the confidence level is above (or equal to or above) the threshold, as shown in step 316, the method 300 may include the processing module using the plurality of peaks to determine an instantaneous heart rate of the user. On the other hand, as shown in step 320, the method 300 may include, based on a determination that the confidence level associated with the RR interval is equal to or below the predetermined threshold, the processing module executing one or more computer-executable instructions associated with the frequency analysis algorithm to determine an instantaneous heart rate of the user. The confidence threshold may be dynamically set based on previous confidence levels.

In some embodiments, as shown in step 318 and 322, the method 300 may include the processing module determining a heart rate variability of the user based on the sequence of the instantaneous heart rates.

The system may include a display device configured to render a user interface for displaying the sequence of the instantaneous heart rates of the user, the RR intervals and/or the heart rate variability determined by the processing module. The system may include a storage device configured to store the sequence of the instantaneous heart rates, the RR intervals and/or the heart rate variability determined by the processing module.

In one aspect, the system may switch between different analytical techniques for determining a heart rate such as a statistical technique for detecting a heart rate and a frequency domain technique for detecting a heart rate. These two different modes have different advantages in terms of accuracy, processing efficiency, and information content, and as such may be useful at different times and under different conditions. Rather than selecting one such mode or technique as an attempted optimization, the system may usefully switch back and forth between these differing techniques, or other analytical techniques, using a predetermined criterion. An exemplary statistical technique employs probabilistic peak detection. An exemplary frequency analysis algorithm used in an implementation isolates the highest frequency components of the optical data, checks for harmonics common in both the accelerometer data and the optical data, and performs filtering of the optical data. This latter algorithm may, for example, take as input raw analog signals from the accelerometer (3-axis) and pulse sensors, and output heart rate values or beats per minute (BPM) for a given period of time related to the window of the spectrogram.

The exemplary wearable system may compute heart rate variability (HRV) to obtain an understanding of the recovery status of the body. These values may be captured right before a user awakes or when the user is not moving, in both cases photoplethysmography (PPG) variability yielding equivalence to the ECG HRV. HRV is traditionally measured using an ECG machine and obtaining a time series of RR intervals. Because an exemplary wearable system utilizes photoplethysmography (PPG), it does not obtain the electric signature from the heart beats; instead, the peaks in the obtained signal correspond to arterial blood volume. At rest, these peaks are directly correlated with cardiac cycles, which enables the calculation of HRV via analyzing peak-to-peak intervals (the PPG analog of RR intervals). It has been demonstrated in medical literature that these peak-to-peak intervals, the "PPG variability," is identical to ECG HRV while at rest.

An exemplary system may include a processing module that is configured to automatically adjust one or more operational characteristics of the light emitters and/or the light detectors to minimize power consumption while ensuring that all heart beats of the user are reliably and continuously detected. The operational characteristics may include, but are not limited to, a frequency of light emitted by the light emitters, the number of light emitters activated, a duty cycle of the light emitters, a brightness of the light emitters, a sampling rate of the light detectors, and the like. The processing module may adjust the operational characteristics based on one or more signals or indicators obtained or derived from one or more sensors in the system including, but not limited to, a motion status of the user, a sleep status of the user, historical information on the user's physiological and/or habits, an environmental or contextual condition (e.g., ambient light conditions), a physical characteristic of the user (e.g., the optical characteristics of the user's skin), and the like.

In one embodiment, the processing module may receive data on the motion of the user using, for example, an accelerometer. The processing module may process the motion data to determine a motion status of the user which indicates the level of motion of the user, for example, exercise, light motion (e.g., walking), no motion or rest, sleep, and the like. The processing module may adjust the duty cycle of one or more light emitters and the corresponding sampling rate of the one or more light detectors based on the motion status. For example, light emitters for PPG may be activated at a duty cycle ranging from about 1% to about 100%. In another example, the light emitters may be activated at a duty cycle ranging from about 20% to about 50% to minimize power consumption. Certain exemplary sampling rates of the light detectors may range from about 50 Hz to about 1000 Hz, but are not limited to these exemplary rates. Certain non-limiting sampling rates are, for example, about 100 Hz, 200 Hz, 500 Hz, and the like.

In one non-limiting example, the light detectors may sample continuously when the user is performing an exercise routine so that the error standard deviation is kept within 5 beats per minute (BPM). When the user is at rest, the light detectors may be activated for about a 1% duty cycle-10 milliseconds each second (i.e., 1% of the time) so that the error standard deviation is kept within 5 BPM (including an error standard deviation in the heart rate measurement of 2 BPM and an error standard deviation in the heart rate changes between measurement of 3 BPM). When the user is in light motion (e.g., walking), the light detectors may be activated for about a 10% duty cycle-100 milliseconds each second (i.e., 10% of the time) so that the error standard deviation is kept within 6 BPM (including an error standard deviation in the heart rate measurement of 2 BPM and an error standard deviation in the heart rate changes between measurement of 4 BPM).

The processing module may adjust the brightness of one or more light emitters by adjusting the current supplied to the light emitters. For example, a first level of brightness may be set by current ranging between about 1 mA to about 10 mA, but is not limited to this exemplary range. A second higher level of brightness may be set by current ranging from about 11 mA to about 30 mA, but is not limited to this exemplary range. A third higher level of brightness may be set by current ranging from about 80 mA to about 120 mA, but is not limited to this exemplary range. In one non-limiting example, first, second and third levels of brightness may be set by current of about 5 mA, about 20 mA and about 100 mA, respectively.

Shorter-wavelength LEDs may require more power than is required by other types of heart rate sensors, such as, a piezo-sensor or an infrared sensor. Therefore, an exemplary wearable system may provide and use a unique combination of sensors-one or more light detectors for periods where motion is expected and one or more piezo and/or infrared sensors for low motion periods (e.g., sleep)-to save battery life. Certain other embodiments of a wearable system may exclude piezo-sensors and/or infrared sensors.

For example, upon determining that the motion status indicates that the user is at a first higher level of motion (e.g., exercising), one or more light emitters may be activated to emit light at a first wavelength. Upon determining that the motion status indicates that the user is at a second lower level of motion (e.g., at rest), non-light based sensors may be activated. The threshold levels of motion that trigger adjustment of the type of sensor may be based on one or more factors including, but are not limited to, skin properties, ambient light conditions, and the like.

The system may determine the type of sensor to use at a given time based on the level of motion (e.g., via an accelerometer) and whether the user is asleep (e.g., based on movement input, skin temperature and heart rate). Based on a combination of these factors the system selectively chooses which type of sensor to use in monitoring the heart rate of the user. Common symptoms of being asleep are periods of no movement or small bursts of movement (such as shifting in bed), lower skin temperature (although it is not a dramatic drop from normal), drastic GSR changes, and heart rate that is below the typical resting heart rate when the user is awake. These variables depend on the physiology of a person and thus a machine learning algorithm is trained with user-specific input to determine when he/she is awake/asleep and determine from that the exact parameters that cause the algorithm to deem someone asleep.

In an exemplary configuration, the light detectors may be positioned on the underside of the wearable system and all of the heart rate sensors may be positioned adjacent to each other. For example, the low power sensor(s) may be adjacent to the high power sensor(s) as the sensors may be chosen and placed where the strongest signal occurs. In one example configuration, a 3-axis accelerometer may be used that is located on the top part of the wearable system. In some embodiments, an operational characteristic of the microprocessor may be automatically adjusted to minimize power consumption. This adjustment may be based on a level of motion of the user's body.

More generally, the above description contemplates a variety of techniques for sensing conditions relating to heart rate monitoring or related physiological activity either directly (e.g., confidence levels or accuracy of calculated heart rate) or indirectly (e.g., motion detection, temperature). However measured, these sensed conditions can be used to intelligently select from among a number of different modes, including hardware modes, software modes, and combinations of the foregoing, for monitoring heart rate based on, e.g., accuracy, power usage, detected activity states, and so forth. Thus there is disclosed herein techniques for selecting from among two or more different heart rate monitoring modes according to a sensed condition.

Exemplary embodiments provide an analytics system for providing qualitative and quantitative monitoring of a user's body, health and physical training. The analytics system may be implemented in computer-executable instructions encoded on one or more non-transitory computer-readable media. The analytics system may rely on and use continuous data on one or more physiological parameters including, but not limited to, heart rate. The continuous data used by the analytics system may be obtained or derived from an exemplary physiological measurement system disclosed herein, or may be obtained or derived from a derived source or system, for example, a database of physiological data. In some embodiments, the analytics system may compute, store and display one or more indicators or scores relating to the user's body, health and physical training including, but not limited to, an intensity score and a recovery score. The scores may be updated in real-time and continuously or at specific time periods, for example, the recovery score may be determined every morning upon waking up, the intensity score may be determined in real-time or after a workout routine or for an entire day.

In certain exemplary embodiments, a fitness score may be automatically determined based on the physiological data of two or more users of exemplary wearable systems.

An intensity score or indicator may provide an accurate indication of the cardiovascular intensities experienced by the user during a portion of a day, during the entire day or during any desired period of time (e.g., during a week or month). The intensity score may be customized and adapted for the unique physiological properties of the user and takes into account, for example, the user's age, gender, anaerobic threshold, resting heart rate, maximum heart rate, and the like. If determined for an exercise routine, the intensity score provides an indication of the cardiovascular intensities experienced by the user continuously throughout the routine. If determined for a period of including and beyond an exercise routine, the intensity score provides an indication of the cardiovascular intensities experienced by the user during the routine and also the activities the user performed after the routine (e.g., resting on the couch, active day of shopping) that may affect their recovery or exercise readiness.

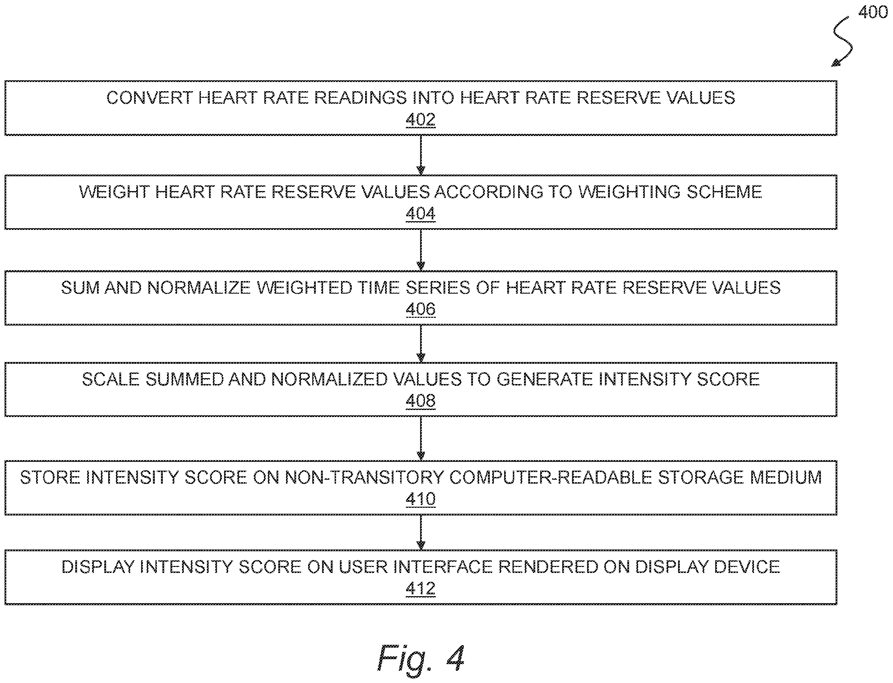

Fig. 4 is a flow chart illustrating an exemplary method of determining an intensity score. In exemplary embodiments, the intensity score may be calculated based on the user's heart rate reserve (HRR) as detected continuously throughout the desired time period, for example, throughout the entire day. In one embodiment, the intensity score may be an integral sum of the weighted HRR detected continuously throughout the desired time period.

As shown in step 402, the method 400 may include converting continuous heart rate readings to HRR values. A time series of heart rate data used in step 402 may be denoted as:

$$H \in T$$

A time series of HRR measurements, v(t), may be defined in the following expression in which MHR is the maximum heart rate and RHR is the resting heart rate of the user:

$$v(t) = \frac{H(t) - RHR}{MHR - RHR}$$

As shown in step 404, the method 400 may include the HRR values are weighted according to a suitable weighting scheme. Cardiovascular intensity, indicated by an intensity score, may be defined in the following expression in which w is a weighting function of the HRR measurements:

$$I(t_o, t_1) = \int_{t_0}^{t_1} w\big(v(t)\big) dt$$

As shown in step 406, the method 400 may include summing and normalizing the weighted time series of HRR values.

$$I_t = \int_T w\big(v(t)\big) dt \leq w(1)|T|$$

Thus, the weighted sum may be normalized to the unit interval, i.e., [0, 1]

$$N_T = \frac{I_T}{w(1) \cdot 24hr}$$

As shown in step 408, the method 400 may include scaling the summed and normalized values to generate user-friendly intensity score values. That is, the unit interval may be transformed to have any desired distribution in a scale (e.g., a scale including 21 points from 0 to 21), for example, arctangent, sigmoid, sinusoidal, and the like. In certain distributions, the intensity values may increase at a linear rate along the scale, and in others, at the highest ranges the intensity values may increase at more than a linear rate to indicate that it is more difficult to climb in the scale toward the extreme end of the scale. In some embodiments, the raw intensity scores may be scaled by fitting a curve to a selected group of "canonical" exercise routines that are predefined to have particular intensity scores.

In one embodiment, monotonic transformations of the unit interval may be achieved to transform the raw HRR values to user-friendly intensity scores. An exemplary scaling scheme, expressed as $f: [0, 1] \rightarrow [0, 1]$, may be performed using the following function:

$$(x, N, p) = 0.5 \left( \frac{arctan(N(x-p))}{\pi / 2} + 1 \right)$$

To generate an intensity score, the resulting value may be multiplied by a number based on the desired scale of the intensity score. For example, if the intensity score is graduated from zero to 21, then the value may be multiplied by 21.

As shown in step 410, the method 400 may include storing the intensity score values on a non-transitory storage medium for retrieval, display and usage.

As shown in step 412, the method 400 may include displaying the intensity score values on a user interface rendered on a visual display device. The intensity score values may be displayed as numbers and/or with the aid of graphical tools, e.g., a graphical display of the scale of intensity scores with current score, and the like. In some embodiments, the intensity score may be indicated by audio.

As shown in step 414, the method 400 may include displaying the intensity score values along with one or more quantitative or qualitative pieces of information on the user. The one or more pieces of information may include, but are not limited to, whether the user has exceeded his/her anaerobic threshold, the heart rate zones experienced by the user during an exercise routine, how difficult an exercise routine was in the context of the user's training, the user's perceived exertion during an exercise routine, whether the exercise regimen of the user should be automatically adjusted (e.g., made easier if the intensity scores are consistently high), whether the user is likely to experience soreness the next day and the level of expected soreness, characteristics of the exercise routine (e.g., how difficult it was for the user, whether the exercise was in bursts or activity, whether the exercise was tapering, etc.), and the like. In one embodiment, the analytics system may automatically generate, store and display an exercise regimen customized based on the intensity scores of the user.

Step 406 may use any of a number of exemplary static or dynamic weighting schemes that enable the intensity score to be customized and adapted for the unique physiological properties of the user. In one exemplary static weighting scheme, the weights applied to the HRR values are based on static models of a physiological process. The human body employs different sources of energy with varying efficiencies and advantages at different HRR levels. For example, at the anaerobic threshold (AT), the body shifts to anaerobic respiration in which the cells produce two adenosine triphosphate (ATP) molecules per glucose molecule, as opposed to 36 at lower HRR levels. At even higher HRR levels, there is a further subsequent threshold (CPT) at which creatine triphosphate (CTP) is employed for respiration with even less efficiency.

In order to account for the differing levels of cardiovascular exertion and efficiency at the different HRR levels, in one embodiment, the possible values of HRR may be divided into a plurality of categories, sections or levels (e.g., three) dependent on the efficiency of cellular respiration at the respective categories. The HRR parameter range may be divided in any suitable manner, such as, piecewise, including piecewise-linear, piecewise-exponential, and the like. An exemplary piecewise-linear division of the HRR parameter range enables weighting each category with strictly increasing values. This scheme captures an accurate indication of the cardiovascular intensity experienced by the user because it is more difficult to spend time at higher HRR values, which suggests that the weighting function should increase at the increasing weight categories.

In one non-limiting example, the HRR parameter range may be considered a range from zero (0) to one (1) and divided into categories with strictly increasing weights. In one example, the HRR parameter range may be divided into a first category of a zero HRR value and may assign this category a weight of zero; a second category of HRR values falling between zero (0) and the user's anaerobic threshold (AT) and may assign this category a weight of one (1); a third category of HRR values falling between the user's anaerobic threshold (AT) and a threshold at which the user's body employs creatine triphosphate for respiration (CPT) and may assign this category a weight of 18; and a fourth category of HRR values falling between the creatine triphosphate threshold (CPT) and one (1) and may assign this category a weight of 42, although other numbers of HRR categories and different weight values are possible. That is, in this example, the weights are defined as:

$$w(v) = \begin{cases} 0 & : v = 0 \\ 1 & : v \in (0, AT] \\ 18 & : v \in (AT, CPT] \\ 42 & : v \in (CPT, 1] \end{cases}$$

In another exemplary embodiment of the weighting scheme, the HRR time series may be weighted iteratively based on the intensity scores determined thus far (e.g., the intensity score accrued thus far) and the path taken by the HRR values to get to the present intensity score. In another exemplary embodiment of the weighting scheme, a predictive approach may be used by modeling the weights or coefficients to be the coefficient estimates of a logistic regression model. One of ordinary skill in the art will recognize that two or more aspects of any of the disclosed weighting schemes may be applied separately or in combination in an exemplary method for determining an intensity score.

In one aspect, heart rate zones may quantify the intensity of workouts by weighing and comparing different levels of heart activity as percentages of maximum heart rate. Analysis of the amount of time an individual spends training at a certain percentage of his/her MHR may reveal his/her state of physical exertion during a workout. This intensity, developed from the heart rate zone analysis, motion, and activity, may then indicate his/her need for rest and recovery after the workout, e.g., to minimize delayed onset muscle soreness (DOMS) and prepare him/her for further activity. As discussed above, MHR, heart rate zones, time spent above the anaerobic threshold, and HRV in RSA (Respiratory Sinus Arrhythmia) regions-as well as personal information (gender, age, height, weight, etc.) may be utilized in data processing.

A recovery score or indicator may provide an accurate indication of the level of recovery of a user's body and health after a period of physical exertion. The human autonomic nervous system controls the involuntary aspects of the body's physiology and is typically subdivided into two branches: parasympathetic (deactivating) and sympathetic (activating). Heart rate variability (HRV), i.e., the fluctuation in inter-heartbeat interval time, is a commonly studied result of the interplay between these two competing branches. Parasympathetic activation reflects inputs from internal organs, causing a decrease in heart rate. Sympathetic activation increases in response to stress, exercise and disease, causing an increase in heart rate. For example, when high intensity exercise takes place, the sympathetic response to the exercise persists long after the completion of the exercise. When high intensity exercise is followed by insufficient recovery, this imbalance lasts typically until the next morning, resulting in a low morning HRV. This result should be taken as a warning sign as it indicates that the parasympathetic system was suppressed throughout the night. While suppressed, normal repair and maintenance processes that ordinarily would occur during sleep were suppressed as well. Suppression of the normal repair and maintenance processes results in an unprepared state for the next day, making subsequent exercise attempts more challenging.

The recovery score may be customized and adapted for the unique physiological properties of the user and takes into account, for example, the user's heart rate variability (HRV), resting heart rate, sleep quality and recent physiological strain (indicated, in one example, by the intensity score of the user). In one exemplary embodiment, the recovery score may be a weighted combination of the user's heart rate variability (HRV), resting heart rate, sleep quality indicated by a sleep score, and recent strain (indicated, in one example, by the intensity score of the user). In an exemplar, the sleep score combined with performance readiness measures (such as, morning heart rate and morning heart rate variability) may provide a complete overview of recovery to the user. By considering sleep and HRV alone or in combination, the user can understand how exercise-ready they are each day and understand how they arrived at the exercise-readiness score each day, for example, whether a low exercise-readiness score is a predictor of poor recovery habits or an inappropriate training schedule. This insight aids the user in adjusting his/her daily activities, exercise regimen and sleeping schedule therefore obtain the most out of his/her training.

In some cases, the recovery score may take into account perceived psychological strain experienced by the user. In some cases, perceived psychological strain may be detected from user input via, for example, a questionnaire on a mobile device or web application. In other cases, psychological strain may be determined automatically by detecting changes in sympathetic activation based on one or more parameters including, but not limited to, heart rate variability, heart rate, galvanic skin response, and the like.

With regard to the user's HRV used in determining the recovery score, suitable techniques for analyzing HRV include, but are not limited to, time-domain methods, frequency-domain methods, geometric methods and non-linear methods. In one embodiment, the HRV metric of the root-mean-square of successive differences (RMSSD) of RR intervals may be used. The analytics system may consider the magnitude of the differences between 7-day moving averages and 3-day moving averages of these readings for a given day. Other embodiments may use Poincaré Plot analysis or other suitable metrics of HRV.

The recovery score algorithm may take into account RHR along with history of past intensity and recovery scores.

With regard to the user's resting heart rate, moving averages of the resting heart rate may be analyzed to determine significant deviations. Consideration of the moving averages is important since day-to-day physiological variation is quite large even in healthy individuals. Therefore, the analytics system may perform a smoothing operation to distinguish changes from normal fluctuations.

Although an inactive condition, sleep is a highly active recovery state during which a major portion of the physiological recovery process takes place. Nonetheless, a small, yet significant, amount of recovery can occur throughout the day by rehydration, macronutrient replacement, lactic acid removal, glycogen re-synthesis, growth hormone production and a limited amount of musculoskeletal repair. In assessing the user's sleep quality, the analytics system may generate a sleep score using continuous data collected by an exemplary physiological measurement system regarding the user's heart rate, skin conductivity, ambient temperature and accelerometer/gyroscope data throughout the user's sleep. Collection and use of these four streams of data enable an understanding of sleep previously only accessible through invasive and disruptive over-night laboratory testing. For example, an increase in skin conductivity when ambient temperature is not increasing, the wearer's heart rate is low, and the accelerometer/gyroscope shows little motion, may indicate that the wearer has fallen asleep. The sleep score indicates and is a measure of sleep efficiency (how good the user's sleep was) and sleep duration (if the user had sufficient sleep). Each of these measures may be determined by a combination of physiological parameters, personal habits and daily stress/strain (intensity) inputs. The actual data measuring the time spent in various stages of sleep may be combined with the wearer's recent daily history and a longer-term data set describing the wearer's personal habits to assess the level of sleep sufficiency achieved by the user. The sleep score may be designed to model sleep quality in the context of sleep duration and history. It thus takes advantage of the continuous monitoring nature of the exemplary physiological measurement systems disclosed herein by considering each sleep period in the context of biologically-determined sleep needs, pattern-determined sleep needs and historically-determined sleep debt.

The recovery and sleep score values may be stored on a non-transitory storage medium for retrieval, display and usage. The recovery and/or sleep score values may be, in some embodiments, displayed on a user interface rendered on a visual display device. The recovery and/or sleep score values may be displayed as numbers and/or with the aid of graphical tools, e.g., a graphical display of the scale of recovery scores with current score, and the like. In some embodiments, the recovery and/or sleep score may be indicated by audio. The recovery score values may be, in some embodiments, displayed along with one or more quantitative or qualitative pieces of information on the user including, but not limited to, whether the user has recovered sufficiently, what level of activity the user is prepared to perform, whether the user is prepared to perform an exercise routine a particular desired intensity, whether the user should rest and the duration of recommended rest, whether the exercise regimen of the user should be automatically adjusted (e.g., made easier if the recovery score is low), and the like. In one embodiment, the analytics system may automatically generate, store and display an exercise regimen customized based on the recovery scores of the user alone or in combination with the intensity scores.

As discussed above, the sleep performance metric may be based on parameters like the number of hours of sleep, sleep onset latency, and the number of sleep disturbances. In this manner, the score may compare a tactical athlete's duration and quality of sleep in relation to the tactical athlete's evolving sleep need (e.g., a number of hours based on recent strain, habitual sleep need, signs of sickness, and sleep debt). By way of example, a soldier may have a dynamically changing need for sleep, and it may be important to consider the total hours of sleep in relation to the amount of sleep that may have been required. By providing an accurate sensor for sleep and sleep performance, an aspect may evaluate sleep in the context of the overall day and lifestyle of a specific user.

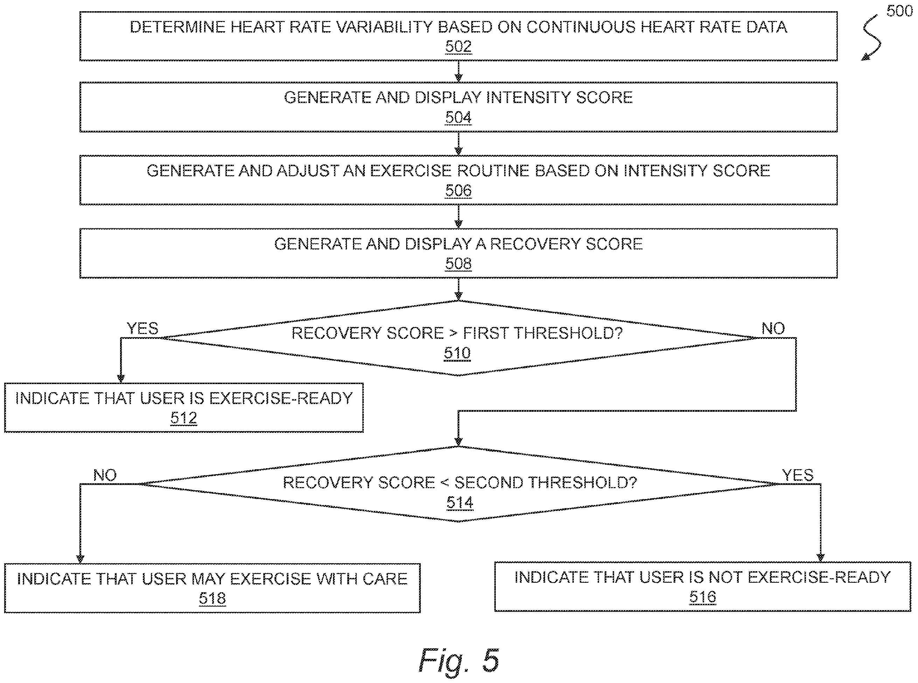

Fig. 5 is a flow chart illustrating an exemplary method by which a user may use intensity and recovery scores. As shown in step 502, the method 500 may include the wearable physiological measurement system determining heart rate variability (HRV) measurements based on continuous heart rate data collected by an exemplary physiological measurement system. In some cases, it may take the collection of several days of heart rate data to obtain an accurate baseline for the HRV.

As shown in step 504, the method 500 may include the analytics system generating and displaying an intensity score for an entire day or an exercise routine. In some cases, the analytics system may display quantitative and/or qualitative information corresponding to the intensity score.

As shown in step 506, the method 500 may include the analytics system automatically generating or adjusting an exercise routine or regimen based on the user's actual intensity scores or desired intensity scores. For example, based on inputs of the user's actual intensity scores, a desired intensity score (that is higher than the actual intensity scores) and a first exercise routine currently performed by the user (e.g., walking), the analytics system may recommend a second different exercise routine that is typically associated with higher intensity scores than the first exercise routine (e.g., running).

As shown in step 508, the method 500 may include, at any given time during the day (e.g., every morning), generating and displaying a recovery score. In some cases, the analytics system may display quantitative and/or qualitative information corresponding to the intensity score. For example, as shown in step 510, the method 500 may include determining if the recovery is greater than (or equal to or greater than) a first predetermined threshold (e.g., about 60% to about 80% in some examples) that indicates that the user is recovered and is ready for exercise. If this is the case, as shown in step 512, the method 500 may include indicating that the user is ready to perform an exercise routine at a desired intensity or that the user is ready to perform an exercise routine more challenging than the past day's routine. Otherwise, as shown in step 514, the method 500 may include determining if the recovery is lower than (or equal to or lower than) a second predetermined threshold (e.g., about 10% to about 40% in some examples) that indicates that the user has not recovered. If this is the case, as shown in step 516, the method 500 may include indicating that the user should not exercise and should rest for an extended period. The analytics system may, in some cases, method 500 may include specifying a duration of recommended rest. Otherwise, as shown in step 518, the method 500 may include indicating that the user may exercise according to his/her exercise regimen while being careful not to overexert him/herself. The thresholds may, in some cases, be adjusted based on a desired intensity at which the user desires to exercise. For example, the thresholds may be increased for higher planned intensity scores.

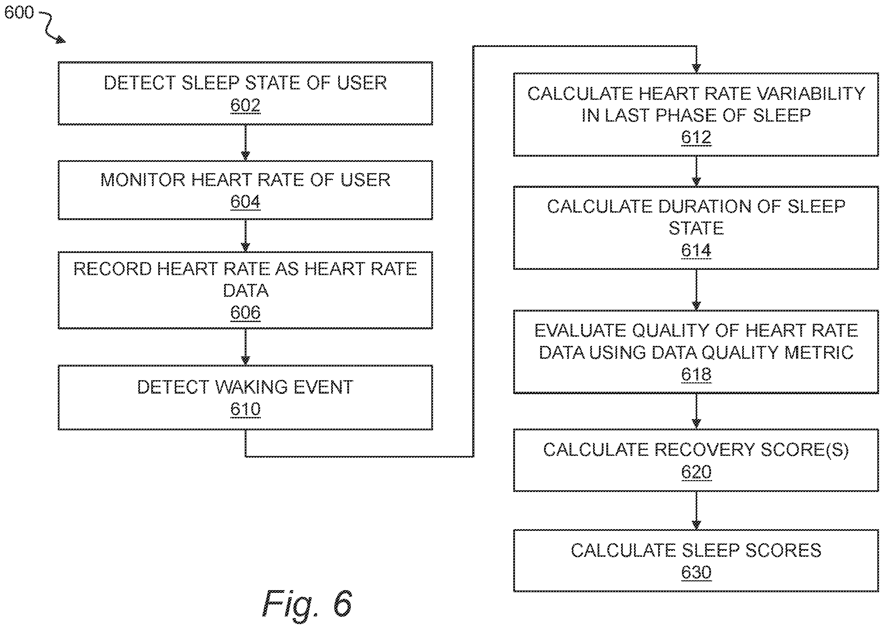

Fig. 6 is a flow chart illustrating a method for detecting heart rate variability in sleep states. The method 600 may be used in cooperation with any of the devices, systems, and methods described herein, such as by operating a wearable, continuous physiological monitoring device to perform the following steps. The wearable, continuous physiological monitoring system may for example include a processor, one or more light emitting diodes, one or more light detectors configured to obtain heart rate data from a user, and one or more other sensors to assist in detecting stages of sleep. In general, the method 600 aims to measure heart rate variability in the last phase of sleep before waking in order to provide a consistent and accurate basis for calculating a physical recovery score.

As shown in step 602, the method 600 may include detecting a sleep state of a user. This may, for example, include any form of continuous or periodic monitoring of sleep states using any of a variety of sensors or algorithms as generally described herein.

Sleep states (also be referred to as "sleep phases," "sleep cycles," "sleep stages," or the like) may include rapid eye movement (REM) sleep, non-REM sleep, or any states/stages included therein. The sleep states may include different phases of non-REM sleep, including Stages 1-3. Stage 1 of non-REM sleep generally includes a state where a person's eyes are closed, but the person can be easily awakened; Stage 2 of non-REM sleep generally includes a state where a person is in light sleep, i.e., where the person's heart rate slows and their body temperature drops in preparation for deeper sleep; and Stage 3 of non-REM sleep generally includes a state of deep sleep, where a person is not easily awakened. Stage 3 is often referred to as delta sleep, deep sleep, or slow wave sleep (i.e., from the high amplitude but small frequency brain waves typically found in this stage). Slow wave sleep is thought to be the most restful form of sleep, which relieves subjective feelings of sleepiness and restores the body.

REM sleep on the other hand typically occurs 1-2 hours after falling asleep. REM sleep may include different periods, stages, or phases, all of which may be included within the sleep states that are detected as described herein. During REM sleep, breathing may become more rapid, irregular and shallow, eyes may jerk rapidly (thus the term "Rapid Eye Movement" or "REM"), and limb muscles may be temporarily paralyzed. Brain waves during this stage typically increase to levels experienced when a person is awake. Also, heart rate, cardiac pressure, cardiac output, and arterial pressure may become irregular when the body moves into REM sleep. This is the sleep state in which most dreams occur, and, if awoken during REM sleep, a person can typically remember the dreams. Most people experience three to five intervals of REM sleep each night.

Homeostasis is the balance between sleeping and waking, and having proper homeostasis may be beneficial to a person's health. Lack of sleep is commonly referred to as sleep deprivation, which tends to cause slower brain waves, a shorter attention span, heightened anxiety, impaired memory, mood disorders, and general mental, emotional, and physical fatigue. Sleep debt (the effect of not getting enough sleep) may result in the diminished abilities to perform high-level cognitive functions. A person's circadian rhythms (i.e., biological processes that display an endogenous, entrainable oscillation of about 24 hours) may be a factor in a person's optimal amount of sleep. Thus, sleep may in general be usefully monitored as a proxy for physical recovery. However, a person's heart rate variability at a particular moment during sleep - during the last phase of sleep preceding a waking event -- can further provide an accurate and consistent basis for objectively calculating a recovery score following a period of sleep.

According to the foregoing, sleep of a user may be monitored to detect various sleep states, transitions, and other sleep-related information. For example, the device may monitor/detect the duration of sleep states, the transitions between sleep states, the number of sleep cycles or particular states, the number of transitions, the number of waking events, the transitions to an awake state, and so forth. Sleep states may be monitored and detected using a variety of strategies and sensor configurations according to the underlying physiological phenomena. For example, body temperature may be usefully correlated to various sleep states and transitions. Similarly, galvanic skin response may be correlated to sweating activity and various sleep states, any of which may also be monitored, e.g., with a galvanic skin response sensor, to determine sleep states. Physical motion can also be easily monitored using accelerometers or the like, which can be used to detect waking or other activity involving physical motion. In another aspect, heart rate activity itself may be used to infer various sleep states and transitions, either alone or in combination with other sensor data. Other sensors may also or instead be used to monitor sleep activity, such as brain wave monitors, pupil monitors, and so forth, although the ability to incorporate these types of detection into a continuously wearable physiological monitoring device may be somewhat limited depending on the contemplated configuration.

As shown in step 604, the method 600 may include monitoring a heart rate of the user substantially continuously with the continuous physiological monitoring system. Continuous heart rate monitoring is described above in significant detail, and the description is not repeated here except to note generally that this may include raw sensor data, heart rate data or peak data, and heart rate variability data over some historical period that can be subsequently correlated to various sleep states and activities.

As shown in step 606, the method 600 may include recording the heart rate as heart rate data. This may include storing the heart rate data in any raw or processed form on the device, or transmitting the data to a local or remote location for storage. In one aspect, the data may be stored as peak-to-peak data or in some other semi-processed form without calculating heart rate variability. This may be useful as a technique for conserving processing resources in a variety of contexts, for example where only the heart rate variability at a particular time is of interest. Data may be logged in some unprocessed or semi-processed form, and then the heart rate variability at a particular point in time can be calculated once the relevant point in time has been identified.

As shown in step 610, the method 600 may include detecting a waking event at a transition from the sleep state of the user to an awake state. It should be appreciated that the waking event may be a result of a natural termination of sleep, e.g., after a full night's rest, or in response to an external stimulus that causes awakening prior to completion of a natural sleep cycle. Regardless of the precipitating event(s), the waking event may be detected via the various physiological changes described above, or using any other suitable techniques. While the emphasis herein is on a wearable, continuous monitoring device, it will be understood that the device may also receive inputs from an external device such as a camera (for motion detection) or an infrared camera (for body temperature detection) that can be used to aid in accurately assessing various sleep states and transitions.

Thus, the wearable, continuous physiological monitoring system may generally detect a waking event using one or more sensors including, for example, one or more of an accelerometer, a galvanic skin response sensor, a light sensor, and so forth. For example, in one aspect, the waking event may be detected using a combination of motion data and heart rate data.

As shown in step 612, the method 600 may include calculating a heart rate variability of the user at a moment in a last phase of sleep preceding the waking event based upon the heart rate data. While a waking event and a history of sleep states are helpful information for assessing recovery, the method 600 described herein specifically contemplates use of the heart rate variability in a last phase of sleep as a consistent foundation for calculating recovery scores for a device user. Thus, step 612 may also include detecting a slow wave sleep period immediately prior to the waking event, or otherwise determining the end of a slow wave or deep sleep episode immediately preceding the waking event.

It will be appreciated that the last phase of sleep preceding a natural waking event may be slow wave sleep. However, where a sleeper is awakened prematurely, this may instead include a last recorded episode of REM sleep or some other phase of sleep immediately preceding the waking event. This moment-the end of the last phase of sleep before waking-is the point at which heart rate variability data provides the most accurate and consistent indicator of physical recovery. Thus, with the appropriate point of time identified, the historical heart rate data (in whatever form) may be used with the techniques described above to calculate the corresponding heart rate variability. It will be further noted that the time period for this calculation may be selected with varying degrees of granularity depending on the ability to accurate detect the last phase of sleep and an end of the last phase of sleep. Thus, for example, the time may be a predetermined amount of time before waking, or at the end of slow wave sleep, or some predetermined amount of time before the end of slow wave sleep is either detected or inferred. In another aspect, an average heart rate variability or similar metric may be determined for any number of discrete measurements within a window around the time of interest.

As shown in step 614, the method 600 may include calculating a duration of the sleep state. The quantity and quality of sleep may be highly relevant to physical recovery, and as such the duration of the sleep state may be used to calculate a recovery score.

As shown in step 618, the method 600 may include evaluating a quality of heart rate data using a data quality metric for a slow wave sleep period, e.g., the slow wave sleep period occurring most recently before the waking event. As noted above, the quality of heart rate measurements may vary over time for a variety of reasons. Thus, the quality of heart rate data may be evaluated prior to selecting a particular moment or window of heart rate data for calculating heart rate variability, and the method 600 may include using this quality data to select suitable values for calculating a recovery score. For example, the method 600 may include calculating the heart rate variability for a window of predetermined duration within the slow wave sleep period having the highest quality of heart rate data according to the data quality metric.

As shown in step 620, the method 600 may include calculating a recovery score for the user based upon the heart rate variability from the last phase of sleep. The calculation may be based on other sources of data. For example, the calculation of recovery score may be based on the duration of sleep, the stages of sleep detected or information concerning the stages (e.g., amount of time in certain stages), information regarding the most recent slow wave sleep period or another sleep period/state, information from the GSR sensor or other sensor(s), and so on. The method 600 may further include calculating additional recovery scores after one or more other waking events of the user for comparison to the previously calculated recovery score. The actual calculation of a discovery score is described in substantial detail above, and this description is not repeated here except to note that the use of a heart rate variability measurement from the last phase of sleep provides an accurate and consistent basis for evaluating the physical recovery state of a user following a period of sleep.

As shown in step 630, the method 600 may include calculating a sleep score and communicating this score to a user.

In one aspect, the sleep score may be a measure of prior sleep performance. For example, a sleep performance score may quantify, on a scale of 0-100, the ratio of the hours of sleep during a particular resting period compared to the sleep needed. On this scale, if a user sleeps six hours and needed eight hours of sleep, then the sleep performance may be calculated as 75%. The sleep performance score may begin with one or more assumptions about needed sleep, based on, e.g., age, gender, health, fitness level, habits, genetics, and so forth and may be adapted to actual sleep patterns measured for an individual over time.

The sleep score may also or instead include a sleep need score or other objective metric that estimates an amount of sleep needed by the user of the device in a next sleep period. In general, the score may be any suitable quantitative representation including, e.g., a numerical value over some predetermined scale (e.g., 0-10, 1-100, or any other suitable scale) or a representation of a number of hours of sleep that should be targeted by the user. In another aspect, the sleep score may be calculated as the number of additional hours of sleep needed beyond a normal amount of sleep for the user.

The score may be calculated using any suitable inputs that capture, e.g., a current sleep deficit, a measure of strain or exercise intensity over some predetermined prior interval, an accounting for any naps or other resting, and so forth. A variety of factors may affect the actual sleep need, including physiological attributes such as age, gender, health, genetics and so forth, as well as daytime activities, stress, napping, sleep deficit or deprivation, and so forth. The sleep deficit may itself be based on prior sleep need and actual sleep performance (quality, duration, waking intervals, etc.) over some historical window. In one aspect, an objective scoring function for sleep need may have a model of the form:

$$\textbf{[0132]} \quad \textit{SleepNeed} = \textit{Baseline} + f_1(\textit{strain}) + f_2(\textit{debt}) - \textit{Naps}$$

In general, this calculation aims to estimate the ideal amount of sleep for best rest and recovery during a next sleep period. When accounting for time falling asleep, periods of brief wakefulness, and so forth, the actual time that should be dedicated to sleep may be somewhat higher, and this may be explicitly incorporated into the sleep need calculation, or left for a user to appropriately manage sleep habits.

In general, the baseline sleep may represent a standard amount of sleep needed by the user on a typical rest day (e.g., with no strenuous exercise or workout). As noted above, this may depend on a variety of factors, and may be estimated or measured for a particular individual in any suitable manner. The strain component, $f_1(\textit{strain})$, may be assessed based on a previous day's physical intensity, and will typically increase the sleep need. Where intensity or strain is measured on an objective scale from 0 to 21, the strain calculation may take the following form, which yields an additional sleep time needed in minutes for a strain, i:

$$f(i) = \frac{1.7}{1 + e^{\frac{17-i}{3.5}}}$$

The sleep debt, $f_2(\textit{debt})$, may generally measure a carryover of needed sleep that was not attained in a previous day. This may be scaled, and may be capped at a maximum, according to individual sleep characteristics or general information about long term sleep deficit and recovery. Naps may also be accounted for directly by correcting the sleep need for any naps that have been taken, or by calculating a nap factor that is scaled or otherwise manipulated or calculated to more accurately track the actual effect of naps on prospective sleep need.

However calculated, the sleep need may be communicated to a user, such as by displaying a sleep need on a wrist-worn physiological monitoring device, or by sending an e-mail, text message or other alert to the user for display on any suitable device.

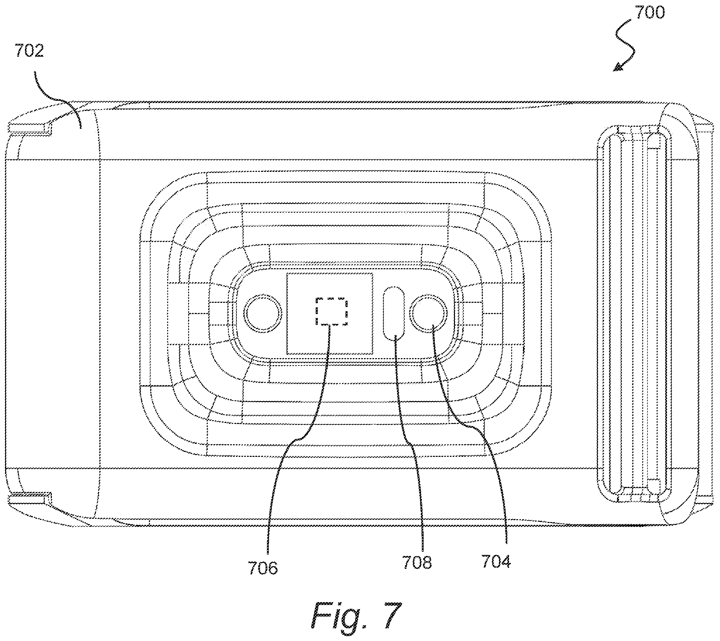

Fig. 7 is a bottom view of a wearable, continuous physiological monitoring device (the side facing a user's skin). As shown in the figure, the wearable, continuous physiological monitoring system 700 includes a wearable housing 702, one or more sensors 704, a processor 706, and a light source 708.

The wearable housing 702 may be configured such that a user can wear a continuous physiological monitoring device as part of the wearable, continuous physiological monitoring system 700. The wearable housing 702 may be configured for cooperation with a strap or the like, e.g., for engagement with an appendage of a user.

The one or more sensors 704 may be disposed in the wearable housing 702. In one aspect, the one or more sensors 704 include a light detector configured to provide data to the processor 706 for calculating a heart rate variability. The one or more sensors 704 may also or instead include an accelerometer configured to provide data to the processor 706 for detecting a sleep state or a waking event. In an implementation, the one or more sensors 704 measure a galvanic skin response of the user.

The processor 706 may be disposed in the wearable housing 702. The processor 706 may be configured to operate the one or more sensors 704 to detect a sleep state of a user wearing the wearable housing 702. The processor 706 may be further configured to monitor a heart rate of the user substantially continuously, and to record the heart rate as heart rate data without calculating a heart rate variability for the user. The processor 706 may also or instead be configured to detect a waking event at a transition from the sleep state of the user to an awake state, and to calculate the heart rate variability of the user at a moment in the last phase of sleep preceding the waking event based upon the heart rate data. The processor 706 may further be configured to calculate a recovery score for the user based upon the heart rate variability from the last phase of sleep.

The light source 708 may be coupled to the wearable housing 702 and controlled by the processor 706. The light source 708 may be directed toward the skin of a user's appendage. Light from the light source 708 may be detected by the one or more sensors 704.

Physiological signals acquired using the various different sensors described herein can be sensitive to conditions under which the physiological signal is obtained. Thus, for example, the physiological signal obtained during certain activities and/or under certain conditions can contain significant amounts of noise, or may have characteristics that vary according to the type of activity or other physical or physiological context. Accordingly, where it is desirable to continuously monitor a physiological signal, it can be advantageous to process the signal using the following techniques in order to reduce or eliminate the negative effects of confounding factors such as motion of the wearable, type of activity, the physical interface with a wearer's skin, weather or other ambient conditions, and so forth.

**Time Domain Processing of Periodic Physiological Signals**

Time domain processing of periodic physiological signals will now be discussed, where any of the following techniques may be used with any of the aforementioned devices, systems, and methods. In particular, the present disclosure includes a robust time domain algorithm for RR-estimation using a state-machine. As a significant advantage, the foregoing techniques facilitate improved time domain peak detection that can be adapted for analysis of heart rate data and extraction of RR intervals and the like for a wider range of subjects, particularly subjects with unusual wave shapes within the QRS complex due to extreme fitness levels, cardiac pathologies, and other unusual cardiac conditions.

These techniques for improved time domain peak detection may be used, for example, to measure heart rate variability (HRV), which may usefully be measured throughout the day, and may be more specifically measured during sleep, e.g., during a particular phase of sleep or the like, in order to evaluate other physical metrics such as strain and recovery. In order for HRV to be estimated accurately, signal processing may be used extract data from the QRS complex, which generally consists of a Q-wave, an R-wave, and an S-wave corresponding to measurable electrical signals during ventricular depolarization. More specifically, HRV is measured based on heartbeat peak-to-peak (RR) interval estimation, where the RR interval is the peak-to-peak distance between two consecutive R-waves.

While a variety of peak detection techniques are known in the art, peak detection can become difficult in certain user-specific contexts such as particular activity types, fitness levels, physiological conditions, and the like that potentially disrupt or mis-shape the characteristic QRS complex used as the basis for peak detection. In order to produce reliable RR estimates over an expanded range of such contexts, a time-domain approach is described herein using state machines having relatively low computational complexity amenable to implementation in a real-time processing environment typical of a wearable physiological monitor. In an aspect, a time domain algorithm uses: (i) a first state machine to process optical data and qualify candidate-peaks for RR-estimation; (ii) a fractional fine-adjustment algorithm/correction to improve resolution of the RR peaks; and (iii) an auxiliary or second state-machine in order to assess if a peak is of "high" or "low" quality. The output of the algorithm may be a set of RR-intervals and a logical flag that indicates if there is "high" or "low" confidence in the RR intervals reported within the buffer. The flag is interchangeably referred to herein as a quality flag, an RR-quality flag, an RRC (RR confidence) flag, and the like, where the flag is generally used to label windows of time domain data as low quality and/or high quality.

The flag, and or an accompanying objective quality metric for confidence in RR estimates (or other heart rate data) may advantageously facilitate additional computational improvements such as (i) improving a decision-tree model, (ii) improving models used for sleep-staging, (iii) extracting pulse-shape features with increased reliability, and the like. Reliable RR-estimation may also or instead enable a system to stage and score sleep accurately. More generally, the techniques described herein can: (i) produce more reliable RR-estimates for a greater range of user-specific contexts including different activity types, fitness levels, and physiological conditions; (ii) be deployed with low computational complexity; (iii) run in a real-time processing environment such as physiological monitoring; (iv) provide an objective measure of data quality for each measured interval; (v) support improved downstream processing of physiological data, and so forth.

In general, photoplethysmography data or other data representative of heart activity may be obtained using optical sensors. The optical signal may be transformed before further time domain processing using a variety of pre-processing techniques such as ambient light cancelation or suppression, bandpass filtering, and so forth. The pre-processed output may then be used as an input to the time domain algorithm as further described herein. Thus, the term "optical data" or "raw data" as used herein may refer to raw signal data from one or more optical transducers, or a pre-processed or otherwise transformed optical signal that is provided as an input to the time domain algorithm. Furthermore, while the following description emphasized processing of photoplethysmography data, the techniques described herein may be usefully employed in other processing environments such as electrocardiography, where a physiological signal of interest varies in response to differing user contexts such as those described above.

In general, a time domain algorithm may include the following steps: (a) optical data buffering; (b) state machine processing, (c) RR-estimation with "fine adjustment"; (d) RR-quality flag construction; and (e) RR rejection due to motion. Each of these steps is explained in more detail below.

## Optical Data Buffering

The optical data may be buffered into a frame that includes a few seconds worth of data sampled at a sampling-rate called "FS" in this disclosure, where the resulting buffer includes "BUF" samples. After each packet of new data is received, the buffer may be updated with a 3/4=75% overlap with a temporally adjacent packet. In particular, the last samples of the buffer may correspond to the newest optical data received, and after each packet is received, the old optical data frame may be shifted by "M" samples to the left. An illustration is provided below.

In this example, the time domain buffer may be characterized by the following vector "b", where $x,y,z,m$ correspond to vectors of size equal to "M" samples each, so that the buffer "b" size is "BUF" samples:

$$b = \{x; y; z; m\}$$

Packet #1. This packet may correspond to time $n$ = 1 second and contain "M" samples worth of optical data denoted by $x[n] = [x^0, x^1, ..., x^{M-1}]$, so that the "BUF" sample buffer is updated as follows:

$$b = \{0; 0; 0; x[n]\}$$

Packet #2. This packet may correspond to time $n$ = 2 seconds and contain "M" samples worth of optical data denoted by $y[n] = [y^0, y^1, ..., y^{M-1}]$, so that the "BUF" sample buffer is updated as follows:

$$b = \{0; 0; x[n-1]; y[n]\}$$

Packet #3. This packet may correspond to time $n$ = 3 seconds and contain "M" samples worth of optical data denoted by $z[n] = [z^0, z^1, ..., z^{M-1}]$, so that the "BUF" sample buffer is updated as follows:

$$b = \{0; x[n-2]; y[n-1]; z[n]\}$$

Packet #4. This packet may correspond to time n = 4 seconds and contain "M" samples worth of optical data denoted by $m[n] = [m^0, m^1, ..., m^{M-1}]$, so that the "BUF" sample buffer is updated as follows:

$$b = \{x[n-3]; y[n-2]; z[n-1]; m[n]\}$$

Packet #5. This packet may correspond to time n = 5 seconds and contain "M" samples worth of optical data denoted by $k[n] = [k^0, k^1, ... , k^{M-1}]$, so that the "BUF" sample buffer is updated as follows:

$$b = \{y[n-3]; z[n-2]; m[n-1]; k[n]\}$$

A number of packets may continue to be processed in a similar manner, where each packet includes 75% of data from the last 3 seconds of a previous packet and 25% new data for the next sequential second of data. This level of overlap has been demonstrated to provide robust characterization of heart activity in practice, however, it will be understood that other packet lengths and overlaps may also or instead be employed.

State Machine Processing of Time Domain Buffer

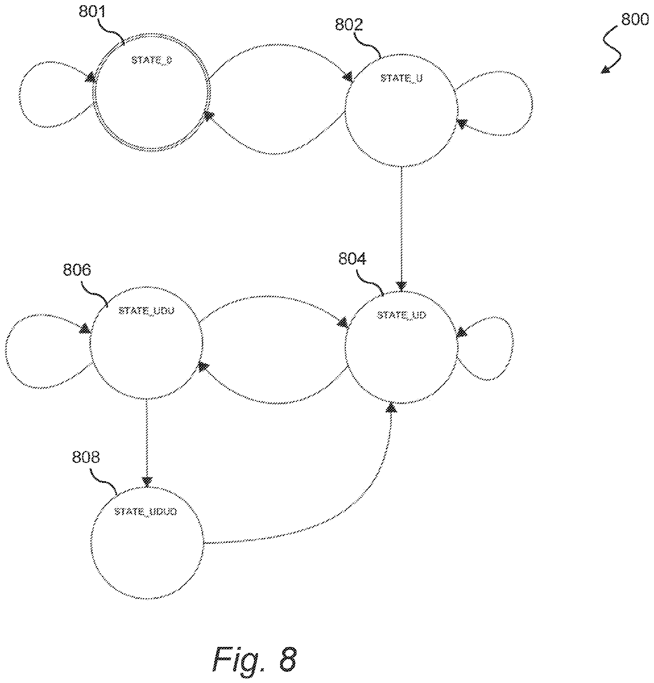

Fig. 8 shows a state machine diagram for processing a buffer of optical data. In particular, for each packet of data received, the buffer of optical data may be processed according to the state machine diagram 800 of Fig. 8. A more detailed algorithm associated with this figure is described below.

For use in the figure, $x = [x^0, x^1, ... , x^{BUF-1}]$ may represent a few seconds worth of optical data sampled at a sample-frequency of "FS", where the resulting buffer includes "BUF" samples. Then $\{x[n]; n \in [0, ..., BUF-1]\}$ corresponds to one of the samples from the time domain buffer.

For every packet of data that is processed, the time domain buffer may start from either $n = 0$ or some other positive integer value depending on whether an RR-interval was previously identified or not. For example, the first packet may be processed by the state machine starting from $n = 0$. If an RR-interval is later identified, then the next time domain buffer may be processed by the state machine starting from another index $n = n_0 > 0$ so that RR-intervals are not duplicated across consecutive packets.

*STATE 0: (Initial State) (Wait for a Positive Edge)* - This represents the initial state 801 in the state machine diagram 800 where the state machine starts looking for R-peaks within a time domain buffer. Once a new packet of data is received, the time domain buffer may be processed starting from either $n = 0$ or some other positive integer value depending on whether an RR-interval was previously identified or not. For example, the very first packet may be processed by the state machine starting from $n = 0$. If an RR-interval is later identified, then the next time domain buffer may be processed by the state machine starting from another index $n = n_0 > 0$ so that RR-intervals are not duplicated across consecutive packets. In either case, for a given packet, processing may start from sample $n = n_0 > 0$ which corresponds to STATE_0.

IF the optical data $x[n]$ exceeds a certain noise-threshold (tuning parameter) AND its previous value $x[n-1]$ was negative THEN a positive edge may be detected and the process may progress to the next state, i.e., STATE_U 802. During the transition, an implementation may (i) save the index associated with the positive edge, and (ii) update a set of summary-statistics and a counter associated with the detected positive pulse. ELSE the process may keep looking for a positive edge event that is reliably over the noise-floor. Thus, the process may return to the same state, namely, STATE_0.

*STATE_U: (First Positive Edge State) (Wait for a Negative Edge)* - At this point, there may be evidence that the process is within the first R-pulse or within a randomly generated pulse. This state aims to resolve this ambiguity.

IF the optical data $x[n]$ exceeds a certain noise-threshold (tuning parameter), THEN the pulse may remain positive, and more information may be collected about it in order to determine if it is an R-pulse or a noisy-pulse. The process may remain in the same state, namely, STATE_U 802. During the transition, a set of summary statistics may continue to be updated, along with a counter associated with the detected positive pulse.

ELSE IF the optical data $x[n]$ is less than a certain noise-threshold (tuning parameter) AND the pulse has been previously positive for a sufficient number of samples (tuning parameter), THEN a negative edge may be detected and the signal may have been positive for a sufficient amount of time so that it is a good first R-pulse candidate (not noise). The process may progress to the next state "STATE_UD" 804. During the transition the process may: (i) save the index associated with the first negative edge, (ii) lock the summary-statistics associated with the first positive pulse and save them for later use, and (iii) update summary statistics and a counter associated with a negative pulse.

ELSE a negative edge may be detected and the signal may have NOT been positive for a sufficient amount of time; this may likely be a noisy-pulse rather than an R-pulse. The process may then go back to the initial state 801 (STATE_0) and start looking for a first R-pulse candidate again. During the transition, the process may (i) reset the positive and negative edge indices to be equal to the current sample "n", and (ii) reset all summary statistics to zero, namely discarding all computations thus far.

*STATE_UD: (First R-Pulse Candidate State) (Wait for a Positive Edge)* - At this point, there may be sufficient evidence that the first pulse identified is an R-pulse rather than noise. This state may aim to look for the beginning of the second R-pulse.

IF the optical data $x[n]$ exceeds a certain noise-threshold (tuning parameter), THEN a positive edge may be detected and the process may progress to the next state STATE_UDU 806. During the transition, the process may: (i) save the index associated with the positive edge, and (ii) update a set of summary-statistics and a counter associated with the detected positive pulse.

ELSE the pulse remains negative and the process may keep waiting for a positive edge. The process may go back to the same state, namely, STATE_UD 804. During the transition, the process may update a counter associated with how many consecutive samples have been negative.

*STATE_UDU: (Second Positive Edge State) (Wait for a Negative Edge)* - At this point, there may be evidence that the process is within the second R-pulse or within a randomly generated pulse. This state aims to resolve this ambiguity.

IF the optical data $x[n]$ exceeds a certain noise threshold (tuning parameter), THEN the pulse may remain positive and more information may be collected about it in order to determine if it is an R-pulse or a noisy-pulse. The process may go back to the same state, namely, STATE_UDU 806. During the transition, the process may update a set of summary statistics and a counter associated with the detected second positive pulse.

ELSE IF the optical data $x[n]$ is less than a certain noise-threshold (tuning parameter) AND the pulse has been previously positive for a sufficient number of sample (tuning parameter), THEN a negative edge may be detected and the signal may have been positive for a sufficient amount of time so that it is a good second R-pulse candidate. The process may progress to the next state STATE_UDUD 808. During the transition, the process may: (i) save the index associated with the second negative edge, and (ii) lock the summary-statistics associated with the second positive pulse and save them for later use.

ELSE a negative edge may be detected and the signal may have NOT been positive for a sufficient amount of time. This is likely a noisy-pulse rather than an R-pulse. The process may go back to STATE_UD 804 and start looking for a second R-pulse candidate again. During the transition, the process may reset all summary statistics associated with the second positive pulse to zero, namely discarding all computations associated with the second positive pulse thus far.

*STATE UDUD: (RR-Pulse Candidate and RR-Quality)* - At this point, there may be sufficient evidence that the first and second pulses identified are both R-pulses rather than noise. This state may aim to compute an RR-interval and provide a metric for RR-confidence (quality). The (previously calculated) summary statistics associated with the first and second positive pulses may be used here.

IF the second-order statistics of the second positive pulse are significantly less than the second-order statistics associated with the first positive pulse (according to a tuning parameter threshold), THEN the second pulse may be physiologically much smaller than the first positive pulse. The second positive pulse may then be ignored and the process may progress back to the state that looks for a second pulse, namely STATE_UD 804. During the transition, the process may reset all summary statistics associated with the second positive pulse to zero, namely discarding all computations associated with the second positive pulse thus far.

ELSE IF the second-order statistics of the first positive pulse are significantly less than the second-order statistics associated with the second positive pulse (according to a tuning parameter threshold), THEN the first pulse physiologically may be much smaller than the second pulse. The first pulse may be set equal to the second pulse and the process may progress back to the state that looks for a second pulse, namely STATE_UD 804. During the transition, the process may: (i) overwrite the start/end indices of the first positive pulse to be equal to those of the second positive pulse, (ii) overwrite the set of summary-statistics and the counter associated with the detected first positive pulse to be equal to those of the second pulse, and (iii) reset all summary statistics associated with the second positive pulse to zero, namely discarding all computations associated with the second positive pulse thus far.

ELSE the first and second pulses have similar area and are considered a physiologically good candidate for RR-estimation. The process may compute a RR-interval calculation and a RR-quality and then transition back to the state that looks for a second pulse, namely STATE_UD 804. This is because there may be more than one RR-interval within a time domain buffer of 4-seconds.

During the transition, the process may compute an RR interval as the difference of the two indices associated with the maximum values of the second and first positive pulses respectively, where this RR interval is reported. Further, during the transition, the process may, IF any of the maximum values associated with the two consecutive positive pulses are less than a noise-threshold (tuning parameter), THEN any RR-intervals identified within the 4-second time domain buffer may be marked/tagged as low-RR-quality. For each of the two consecutive positive pulses identified, the process may perform the processing defined as "assess_peak(.)," which is a separate state machine with the objective of identifying whether the pulse detected is physiologically accurate of not. If it is not physiologically accurate, then any RR-intervals identified within the time domain buffer may be marked/tagged as low-RR-quality. The description of "assess_peak(.)" is provided below in the "State Machine Implementation of 'Assess_Peak'" section of this disclosure.

Following the previous operations, the process may then: (i) overwrite the start/end indices of the first positive pulse to be equal to those of the second positive pulse, (ii) overwrite the set of summary-statistics and the counter associated with the detected first positive pulse to be equal to those of the second pulse, and (iii) reset all summary statistics associated with the second positive pulse to zero, namely discarding all computations associated with the second positive pulse thus far.

Finally, the process may update the starting index for the next packet of data to be equal to $n = n_0 > 0$, where $n_0$ accounts for the start/end indices computed in the current packet of data plus the buffer overlap factor (i.e., 75%). This step may ensure that no duplicate RR-intervals are reported between successive packets due to the overlap associated with the time domain buffer. The state machine on the next packet of data may start processing from index $n = n_0 > 0$.

State Machine Implementation of "Assess_Peak"

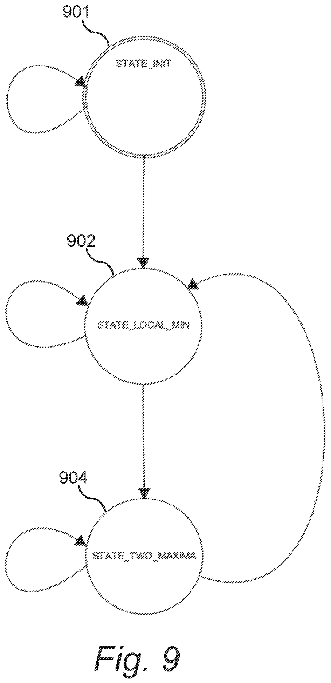

Fig. 9 shows a state machine diagram for identifying accuracy of a pulse. In general, for a given window of interest that captures the beginning and end of a positive pulse (as described above regarding STATE_UDUD), implementations may opt to identify if the pulse is physiologically accurate or not. If it is not physiologically accurate, then any RR-intervals identified within the time domain buffer may be marked/tagged as low RR-quality. The underlying processing may involve traversing through the state machine diagram 900 of Fig. 9, where a detailed "assess_peak" algorithm associated with this figure is outlined below.

In the figure, $x = [x^0, x^1, ... , x^{N-1}]$ may represent a variable window that includes "N" samples worth of optical data. The length of the window "N" may be passed from STATE_UDUD discussed above. "N" may be equal to the difference between the "end" and "start" of the positive pulse that is currently being assessed.

*STATE_INIT: (Initial State) (Searching for a First Local Maximum)* - STATE_INIT 901 may represent the initial state, which represents the starting point of looking for R-peaks within a time domain buffer (as invoked in STATE_UDUD discussed above). Once a new window of time domain data is received, it may be processed starting from "n=0" up to "n=N-1", so that a loop iterates with values $n = \{0, 1, ..., N - 1\}$.

IF the optical data $x[n]$ exceeds a certain noise-threshold (tuning parameter) AND the current sample "$x[n]$" is greater than the previous sample "$x[n - 1]$", THEN a "first" local maximum may have been identified and the process may progress to the next state, STATE_LOCAL_MIN 902. During the transition, the process may store the location and value of the first local maximum so that it can be used later.

ELSE the search for the first local maximum is continued and the process may go back to the same state, namely, STATE_INIT 901.

*STATE_LOCAL_MIN: (Searching for a Local Minimum in between two Local Maxima)* - At this point, the process may have identified the first local maximum and may turn to detecting the first local minimum that occurs right after.

IF the optical data $x[n]$ exceeds a certain noise-threshold (tuning parameter) AND the current sample "$x[n]$" is greater than the previous sample "$x[n - 1]$", THEN a "first" local minimum may have been identified and the process may progress to the next state, STATE_TWO_MAXIMA 904. During the transition, the process may store the location and value of the first local minimum so that it can be used later.

ELSE the search for the first local minimum is continued, and the process goes back to the same state, namely, STATE_LOCAL_MIN 902.

*STATE_TWO_MAXIMA: (Searching for the Second Local Maximum)* - At this point, the process may have identified the first local minimum that occurs right after the first local maximum, and the process may be interested in detecting the second local maximum that occurs right after the first local minimum. This state can set the "low-RR-quality" flag to "True" if a certain number of conditions are met.

IF the optical data $x[n]$ exceeds a certain noise-threshold (tuning parameter) AND the current sample "$x[n]$" is less than the previous sample "$x[n - 1]$", THEN a "second" local maximum may have been identified and the process may start a new search for a local minimum, namely, looping back to state STATE_LOCAL_MIN 902. During the transition, the process may set the "low-RR-quality" flag to "True" if (i) the two local maxima amplitudes are close to each other (based on a tuning parameter threshold), OR (ii) any of the two local maxima amplitudes are close to the local minimum (based on a tuning parameter threshold).

ELSE the search for the second local maximum is continued, and the process may go back to the same state, namely, STATE_TWO_MAXIMA 904.

Prepare for the Next Packet of Data

After the state machine described above completes processing the current time domain buffer of data, an aspect of the present teachings may perform the following computations in order to prepare for the next packet of data that includes the updated time domain buffer. The computations may include updating the noise threshold(s) mentioned in the state machine calculations by updating first-order statistics of the current time domain buffer. The computations may further include updating the starting index for the next packet of data to be equal to $n = n_0 > 0$, where $n_0$ accounts for the start/end indices computed in the current packet of data plus the buffer overlap factor (e.g., 75%). This step may ensure that no duplicate RR-intervals are reported between successive packets due to the overlap associated with the time domain buffer. The state machine on the next packet of data may start processing from index $n = n_0 > 0$. The computations may further include, if the "low-RR-Quality" flag was set inside the state machine STATE_UDUD, then mark the current time domain buffer as "low-RR-Quality" or the like.

Fractional Fine-Adjustment Algorithm

An example of a fractional fine-adjustment algorithm is summarized below, where this algorithm aims to interpolate the location of a maximum in the case where a peak is relatively flat. In this example, the "coarse" peak value may be denoted as "*centerValue*"; the "*centerValue*" may be equal to "*maxVal1*", "*maxVal2*", etc., as computed by the state machine. The "coarse" peak location may be denoted as "*coarseLoc*", which may be centered at "x=0". The "*centerValue*" may be equal to "*maxLoc1*", "*maxLoc2*", etc., as computed by the state machine. Each of these may be centered around zero "x=0". The immediate left and right values relative to the "*centerValue*" may be denoted as "*leftValue*" and "*rightValue*" respectively. The locations associated with "*leftValue*" and "*rightValue*" may be "*x=-1*" and "*x=+1*", respectively.

A quadratic function may be defined as:

$$f(x) = a \cdot x^2 + b \cdot x + c$$

Where:

$$f(x = 0) = centerValue = a \cdot (0)^2 + b \cdot (0) + c \Rightarrow$$

$$c = centerValue$$

$$f(x = -1) = leftValue = a \cdot (-1)^2 + b \cdot (-1) + centerValue \Rightarrow$$

$$a - b = leftValue - centerValue$$

$$f(x = +1) = rightValue = a \cdot (+1)^2 + b \cdot (+1) + centerValue \Rightarrow$$

$$a + b = rightValue$$

So that:

$$a = \frac{rightValue + leftValue - 2 \cdot centerValue}{2}$$

$$b = \frac{rightValue - leftValue}{2}$$

The extremum associated with the quadratic surface may be computed using the first-derivative criterion, namely:

$$f'(x) = 0$$

In addition, based on the definition of "*centerValue*", "*leftValue*", and "*rightValue*", the extremum is guaranteed to correspond to a "maximum".

$$f'(x) = 0 \Rightarrow$$

$$f'(x) = 2 \cdot a \cdot x + b = 0 \Rightarrow$$

$$fineAdjustment = -\frac{b}{2 \cdot a} \Rightarrow$$

$$fineAdjustment = -\frac{leftValue - rightValue}{2 \cdot (rightValue + leftValue - 2 \cdot centerValue)}$$

Finally, the fractionally adjusted peak location as indicated by the third point(s) in the figures described below is produced by adding the "*coarseLoc*" (red-circle) with the "*fineAdjustment*" computed previously, namely:

$$adjustedPeakLocation = coarseLoc + fineAdjustment$$

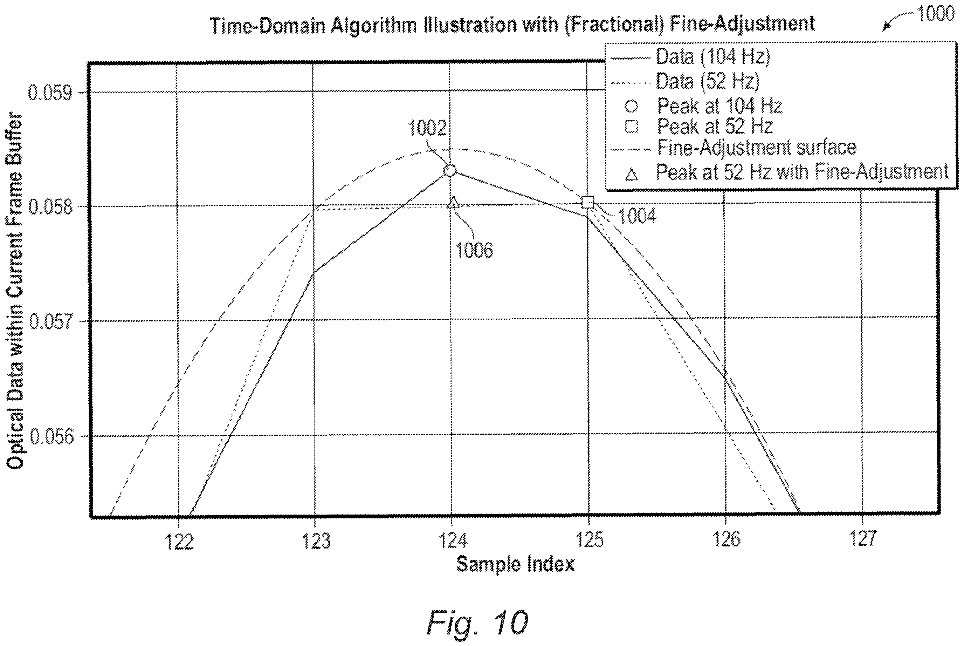

Fig. 10 is a graph including a time series of a time domain buffer of optical data. Specifically, the graph 1000 shows a zoomed-in view of a time series of time domain buffer of optical data showing a first point 1002 corresponding to the accurate location of a peak, a second point 1004 indicating the case of a flat peak, and a third point 1006 corresponding to the result of fractional fine adjustment applied on the second point 1004 so that the accuracy is recovered and is closer to that of the first point 1002.

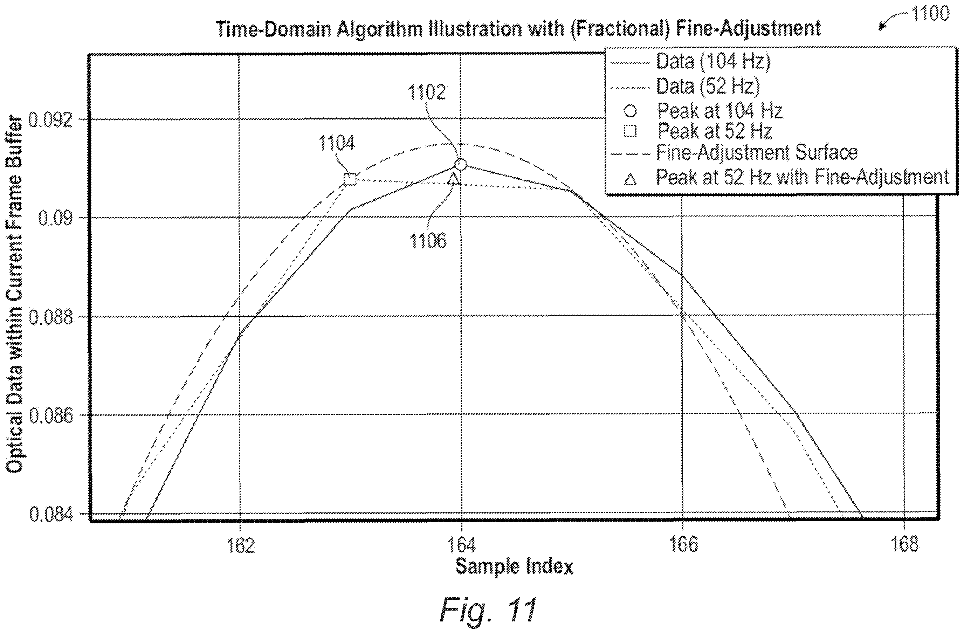

Fig. 11 is a graph including a time series of a time domain buffer of optical data. Specifically, the graph 1100 shows a zoomed-in view of a time series of time domain buffer of optical data showing a first point 1102 corresponding to the accurate location of a peak, a second point 1104 indicating the case of a flat peak, and a third point 1106 corresponding to the result of fractional fine adjustment applied on the second point 1104 so that the accuracy is recovered and is closer to that of the first point 1102.

RR-Interval Computation

The process may continue to use the "*adjustedPeakLocations*" post fractional fine-adjustment for each peak in order to compute RR-intervals. Within a time domain buffer there may exist zero, one, or more RR-intervals, where the formula for an RR-intervals is:

$$RR = (adjustedMaxLoc2 - adjustedMaxLoc1)/SAMPLE\_RATE$$

Where "*SAMPLE_RATE=FS*" corresponds to the sample-rate (FS samples-per-second). The "*maxLoc1*", "*maxLoc2*" values are the coarse peak locations identified by the state-machine, and the term "adjusted" refers to the output of the fractional fine-adjustment algorithm.

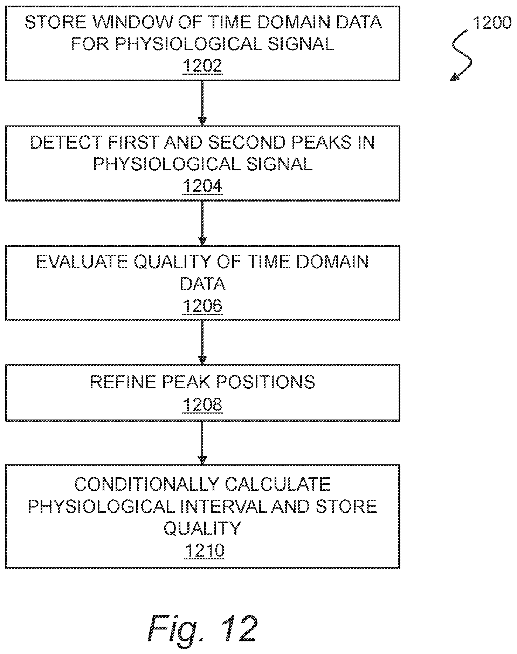

Fig. 12 is a flow chart illustrating a method for time domain processing of periodic physiological signals. In general, estimating physiological data of a user with time domain algorithms may occur while the user is engaged in a wide range of physical activities. Certain scenarios may cause unacceptable levels of noise in the input data. It may be advantageous, then, to determine physiological intervals only when input noise is at an acceptable level. It will be understood that the method 1200 may be implemented using any of the devices and systems disclosed herein, and more specifically, the method 1200 may include steps carried out by the robust time domain algorithm for RR-estimation using a state-machine described in the preceding paragraphs. The method 1200 may be used to assist with sleep tracking performance of a wearable physiological monitoring system.

As shown in step 1202, the method 1200 may include storing a window of time domain data for a physiological signal. The window may include a series of samples of the physiological signal. The physiological signal may be received from one or more sensors of a wearable device such as any of those described herein. By way of example, the physiological signal may include a heart rate signal such as a photoplethysmography signal or an electrocardiography signal. In general, the window of time domain data may be pre-processed, e.g., using any of the techniques described herein to suppress noise, remove outliers, filter non-physiological content, and so forth.

As shown in step 1204, the method 1200 may include detecting a first peak and a second peak in the physiological signal using a time domain process on the series of samples. As described herein, the time domain process may include a first state machine configured to sequentially process the series of samples and identify a first peak and a second peak in the series of samples. To this end, the first state machine may implement a peak detection algorithm. For example, the first state machine may identify a first pulse candidate, which may be a heart rate signal. The first state machine may then conditionally label the first pulse candidate as the first peak when the first pulse candidate is determined to be an R-pulse. The first state machine may then identify a second pulse candidate, which may also be a heart rate signal. The first state machine may then conditionally label the second pulse candidate as the second peak when the second pulse candidate is determined to be an R-pulse. In some embodiments, identifying a pulse candidate may include calculating one or more pulse parameters such as start, end, length, peak, local minimum, global minimum, local maximum, global maximum, and the like.

As shown in step 1206, the method 1200 may include evaluating a quality of the time domain data in the window indicative of how well a time domain shape associated with a peak shape matches an expected peak shape for the physiological signal. As described herein, this evaluation may be performed using a second or auxiliary state machine after R-pulse peaks have been identified. Evaluating the quality of the time domain data may, for example, include characterizing the peak shape by identifying a local minimum located between a first local maximum and a second local maximum with a second state machine. The quality may be indicative of how well a time domain shape associated with a peak matches an expected peak for the physiological signal, and may depend, e.g., on the relative amplitude of the peaks, the location of a minimum between the peaks, the length of the interval between the peaks, and/or any other metrics that can be evaluated by applying a state machine to the time domain data. In some embodiments, evaluating a quality of the time domain data may include conditionally labelling the quality of the time domain data as low-RR-quality when the difference in amplitudes of the first local maximum and the second local maximum are within a predetermined threshold. That is, the quality may be characterized using a marker or flag that is set to low-RR-quality or high-RR-quality.

The method 1200 may also or instead include RR interval quality scoring. That is, in certain implementations, evaluating the quality of the time domain data includes evaluating a first peak quality for the first peak and a second peak quality for the second peak using an objective measure of peak shape. Also, or instead, evaluating the quality of the time domain data may include evaluating local maxima and minima in the time domain data with the second state machine. Thus, in certain aspects, the physiological signal is a heart rate signal, and the quality of the time domain data includes a quality of an RR interval detected within the time domain heart rate signal.

As shown in step 1208, the method 1200 may include locally refining a peak position of each of the first peak and the second peak. Locally refining the peak position may include interpolating a location of a maximum for a number of sequential samples within the time domain data having similar magnitudes. Locally refining the peak position may also or instead include estimating a value of a maximum at a location for the maximum between two of the sequential samples within the time domain data. To this end, estimating the value may include fitting two or more of the number of sequential samples to a quadratic function and calculating the value at a greatest value of the quadratic function. Other fitting techniques may also or instead be used such as a linear interpolation, a triangular approximation, and so forth. While more computationally complex, the quadratic function advantageously captures a more accurate estimate of the location and magnitude of a peak having the approximately parabolic shape characteristic of a typical R-pulse peak.

As shown in step 1210, the method 1200 may include, when the quality exceeds a predetermined threshold, conditionally calculating a physiological interval for the window of time domain data and storing a value based on the quality as a quality flag for the window of time domain data. The physiological interval may be based on a time between the first peak and the second peak.

In certain aspects, the physiological signal includes photoplethysmography data and the physiological interval includes an RR-interval. In such instances, the method 1200 may further include calculating a pulse rate or other heart rate metric(s) based on the RR-interval. In another aspect, the method 1200 may include calculating interval data for a sequence of overlapping windows of the time domain data. This approach may advantageously improve peak detection and improve detection/rejection of low quality data intervals by providing multiple measurements over a time domain interval. The method 1200 may also include storing any suitable accompanying data. For example, the method 1200 may include storing interval data such as summary data (peak-to-peak time, peak value(s), frequency, etc.) and/or raw data for the physiological interval, as well as a quality measure or quality flag as described above. In one aspect, data may be conditionally stored based on the quality, i.e., by storing data only when the quality is above a predetermined threshold.

According to the foregoing, there is further described herein a system comprising a wearable housing, one or more sensors in the wearable housing for capturing physiological data, and a processor in the wearable housing. The processor may be configured by computer executable code to perform the steps of storing a window of time domain data for a physiological signal, the window including a series of samples of the physiological signal, detecting a first peak and a second peak in the physiological signal using a time domain process on the series of samples, evaluating a quality of the time domain data in the window indicative of how well a time domain shape associated with a peak shape matches an expected peak shape for the physiological signal, locally refining a peak position of each of the first peak and the second peak, conditionally calculating the physiological interval when the quality exceeds a predetermined threshold, and storing a value for a quality flag based on the quality.

Evaluating a quality of the time domain data may include identifying the peak shape by identifying a local minimum located between a first local maximum and a second local maximum with a state machine. Locally refining the peak position may include interpolating a location of a maximum for a number of sequential samples within the time domain data having similar magnitudes. Alternatively or in addition, locally refining the peak position may include estimating a value of a maximum at a location for the maximum between two sequential samples of the time domain data. The time domain process may include a state machine configured to sequentially process the series of samples and identify the first peak and the second peak in the series of samples. In one aspect, the state machine may be further configured to perform the steps of identifying a first pulse candidate, wherein the first pulse candidate is a heart rate signal, conditionally labelling the first pulse candidate as the first peak when the first pulse candidate is determined to be an R-pulse, identifying a second pulse candidate, wherein the second pulse candidate is a heart rate signal, and conditionally labelling the second pulse candidate as the second peak when the second pulse candidate is determined to be an R-pulse. In another aspect, the state machine may be further configured to compare second-order statistics of the first peak and the second peak.

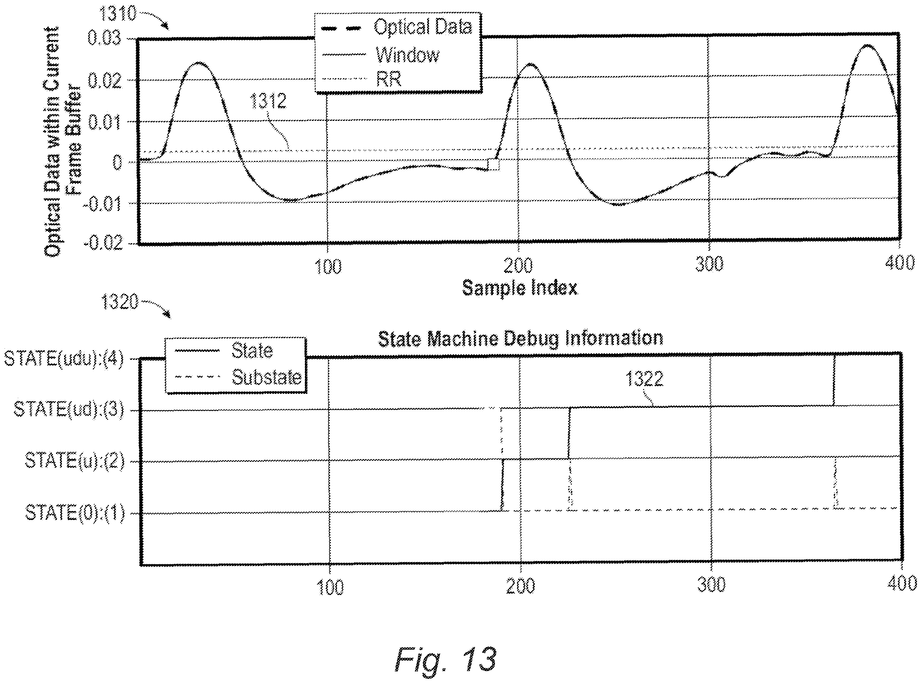

Various examples are shown in Figs. 13-16 and described below for time domain processing as disclosed herein. In these examples, the following criteria will generally apply: each packet of data corresponds to a time domain buffer of optical data (the top graphs in Figs. 13-16); the bottom graphs in Figs. 13-16 correspond to debugging information associated with a state machine as described herein; the solid lines substantially in the top portion of the bottom graphs in Figs. 13-16 represent the active state within the state machine; the solid lines substantially in the bottom portion of the bottom graphs in Figs. 13-16 represent the sub-function called when there is a state transition; the points (if present) on the top graphs in Figs. 13-16 represent peaks identified by the time domain algorithm; a shaded/colored background on the top graphs in Figs. 13-16 represent that the RR-quality is either high or low; no background shading or coloring on the top graphs in Figs. 13-16 represents that no RR-interval was identified during the current time domain buffer; and the horizontal line on the top graphs in Figs. 13-16 represents the value of the noise-threshold used in the calculations of the state machines as described herein.

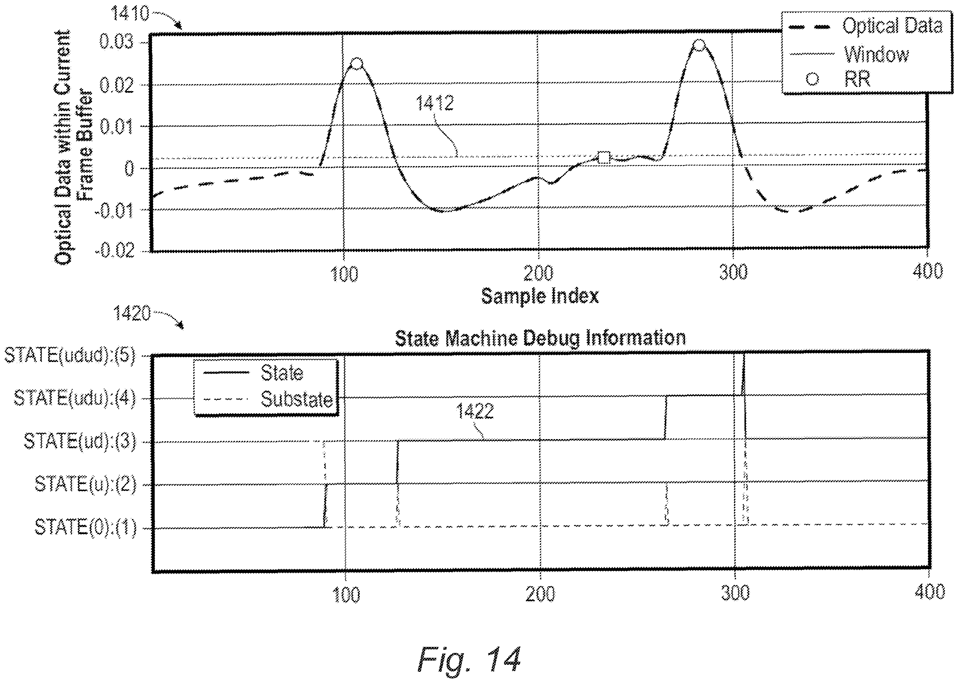

The first example corresponds to Figs. 13 and 14. Fig. 13 shows a first graph including a time domain buffer of optical data and a second graph showing state machine debugging information, and Fig. 14 shows a first graph including a time domain buffer of optical data and a second graph showing state machine debugging information. In particular, Figs. 13 and 14 represent two consecutive packets of data that are received, where each packet of data corresponds to a time domain buffer of optical data in the first graph of the respective figure and debug information associated with a state machine as disclosed herein in the second graph of the respective figure.

Turning to Fig. 13, as shown in the second graph 1320 of that figure, for the current packet, the state machine starts processing from sample $n = n_0 = 180$, and the first state is always "*STATE_0*" for these examples. As shown in the first graph 1310 of Fig. 13, x[n] remains lower than the noise threshold 1312 between samples 180-192, and as shown in the second graph 1320 of Fig. 13, the algorithm is locked into "*STATE_0*" since there is no positive event detected yet as demonstrated by the line representing the active state 1322 between samples 180-192. As shown in the first graph 1310 of Fig. 13, x[n] exceeds the noise threshold 1312 around sample n=192, and as shown in the second graph 1320 of Fig. 13, there is a state transition from "*STATE_0*" to "*STATE_U*" as demonstrated by the line representing the active state 1322 around sample n=192. As shown in the first graph 1310 of Fig. 13, x[n] remains greater than the noise threshold 1312 between samples 192-228, and as shown in the second graph 1320 of Fig. 13, the algorithm is locked into "*STATE_U*" since there is no negative event detected yet as demonstrated by the line representing the active state 1322 between samples 192-228. As shown in the first graph 1310 of Fig. 13, x[n] drops below the noise threshold 1312 around sample n=228, and as shown in the second graph 1320 of Fig. 13, there is a state transition from "*STATE_U*" to "*STATE_UD*" as demonstrated by the line representing the active state 1322 around sample n=228. This means that the duration of the positive pulse detected was sufficient so that it qualifies as an R-wave candidate. As shown in the first graph 1310 of Fig. 13, x[n] remains lower than the noise threshold 1312 between samples 228-366, and as shown in the second graph 1320 of Fig. 13, the algorithm is locked into "*STATE_UD*" since there is no positive event detected yet as demonstrated by the line representing the active state 1322 between samples 228-366. As shown in the first graph 1310 of Fig. 13, x[n] exceeds the noise threshold 1312 around sample n=366, and as shown in the second graph 1320 of Fig. 13, there is a state transition from "*STATE_UD*" to "*STATE_UDU*" as demonstrated by the line representing the active state 1322 around sample n=366. As shown in the first graph 1310 of Fig. 13, x[n] remains greater than the noise threshold 1312 between samples 366-BUF, and as shown in the second graph 1320 of Fig. 13, the algorithm is locked into "*STATE_UDU*" since there is no negative event detected yet as demonstrated by the line representing the active state 1322 between samples 366-BUF. The end of the time domain buffer has thus been reached, as there was not enough data to complete a second pulse and thus no RR-interval is computed for this packet. The search will resume on the next packet (Fig. 14) starting from index $n_0 = 81$ to avoid duplication.

Turning to Fig. 14, as shown in the second graph 1420 of that figure, for the current packet, the state machine starts processing from sample $n = n_0 = 81$ and the first state is always "*STATE_0*". As shown in the first graph 1410 of Fig. 14, x[n] remains lower than the noise threshold 1412 between samples 81-90, and as shown in the second graph 1420 of Fig. 14, the algorithm is locked into "*STATE_0*" since there is no positive event detected yet as demonstrated by the line representing the active state 1422 between samples 81-90. As shown in the first graph 1410 of Fig. 14, x[n] exceeds the noise threshold 1412 around sample n=90, and as shown in the second graph 1420 of Fig. 14, there is a state transition from "*STATE_0*" to "*STATE_U*" as demonstrated by the line representing the active state 1422 around sample n=90. As shown in the first graph 1410 of Fig. 14, x[n] remains greater than the noise threshold 1412 between samples 90-127, and as shown in the second graph 1420 of Fig. 14, the algorithm is locked into "*STATE_U*" since there is no negative event detected yet as demonstrated by the line representing the active state 1422 between samples 90-127. As shown in the first graph 1410 of Fig. 14, x[n] drops below the noise threshold 1412 around sample n=127, and as shown in the second graph 1420 of Fig. 14, there is a state transition from "*STATE_U*" to "*STATE_UD*" as demonstrated by the line representing the active state 1422 around sample n=127. This means that the duration of the positive pulse detected was sufficient so that it qualifies as an R-wave candidate. As shown in the first graph 1410 of Fig. 14, x[n] remains lower than the noise threshold 1412 between samples 127-265, and as shown in the second graph 1420 of Fig. 14, the algorithm is locked into "*STATE_UD*" since there is no positive event detected yet as demonstrated by the line representing the active state 1422 between samples 127-265. As shown in the first graph 1410 of Fig. 14, x[n] exceeds the noise-threshold noise threshold 1412 around sample n=265, and as shown in the second graph 1420 of Fig. 14, there is a state transition from "*STATE_UD*" to "*STATE_UDU*" as demonstrated by the line representing the active state 1422 around sample n=265. As shown in the first graph 1410 of Fig. 14, x[n] remains greater than the noise threshold 1412 between samples 265-305, and as shown in the second graph 1420 of Fig. 14, the algorithm is locked into "*STATE_UDU*" since there is no negative event detected yet as demonstrated by the line representing the active state 1422 between samples 265-305.

As shown in the first graph 1410 of Fig. 14, x[n] drops below the noise threshold 1412 around sample n=305, and as shown in the second graph 1420 of Fig. 14, there is a state transition from "*STATE_UDU*" to "*STATE_UDUD*" as demonstrated by the line representing the active state 1422 around sample n=305. This means that the duration of the positive pulse detected was sufficient so that it qualifies as a second R-wave candidate. At around sample=305, two consecutive R-pulse candidates "*STATE_UDUD*" have been detected. The areas of the two pulses are similar so that the state machine can transition back to state "*STATE_UD*" as demonstrated by the line representing the active state 1422 around sample=305. This allows the computation of an RR-interval indicated by the points at the peaks of the first graph 1410. The RR-interval quality is high and thus the background of the figure is shaded as described above.

As shown in the first graph 1410 of Fig. 14, x[n] remains lower than the noise threshold 1412 between samples 305-400, and as shown in the second graph 1420 of Fig. 14, the algorithm is locked into "*STATE_UD*" since there is no positive event detected yet as demonstrated by the line representing the active state 1422 between samples 305-400. The end of the time domain buffer is reached, and there was enough data to compute one RR-interval and its associated quality was high. The search will resume on the next packet starting from index: $n_0$ = 154 to avoid duplication.

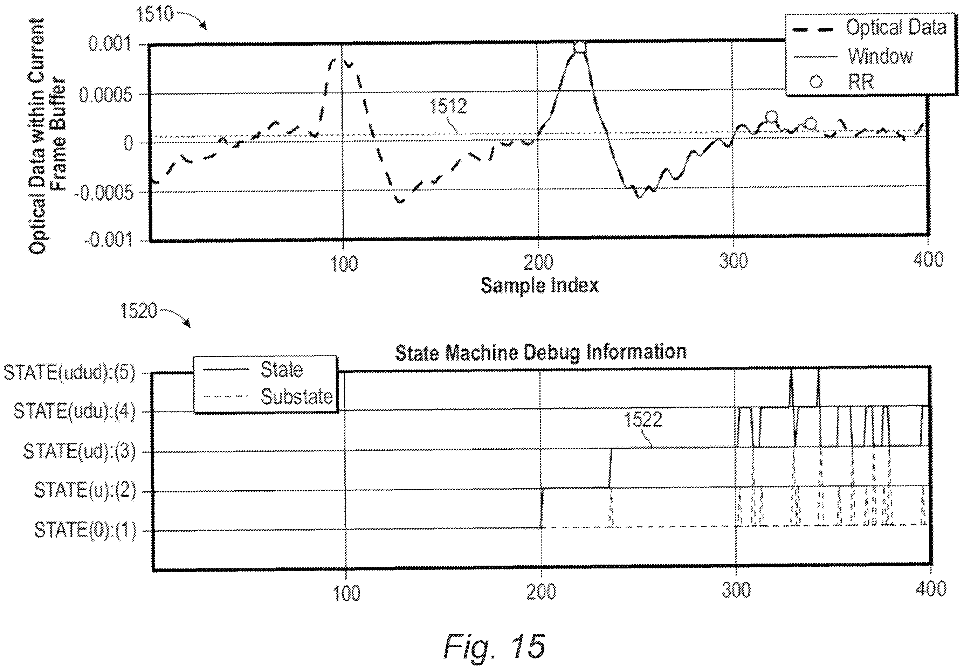

The second example corresponds to Fig. 15. Fig. 15 shows a first graph including a time domain buffer of optical data and a second graph showing state machine debugging information. Specifically, Fig. 15 represents a single packet of data corresponding to a time domain buffer of optical data as shown in the first graph 1510 and debug information associated with the state machine described herein as shown in the second graph 1520.

As shown in the second graph 1520 of Fig. 15, for the current packet, the state machine starts processing from sample $n = n_0$ = 190, and the first state is always "*STATE_0*" as demonstrated by the line representing the active state 1522 at n=190. As shown in the first graph 1510 of Fig. 15, x[n] remains lower than the noise threshold 1512 between samples 190-200, and as shown in the second graph 1520 of Fig. 15, the algorithm is locked into "*STATE_0*" since there is no positive event detected yet as demonstrated by the line representing the active state 1522 between samples 190-200. As shown in the first graph 1510 of Fig. 15, x[n] exceeds the noise threshold 1512 around sample n=200, and as shown in the second graph 1520 of Fig. 15, there is a state transition from "*STATE_0*" to "*STATE_U*" as demonstrated by the line representing the active state 1522 around sample n=200. As shown in the first graph 1510 of Fig. 15, x[n] remains greater than the noise threshold 1512 between samples 200-235, and as shown in the second graph 1520 of Fig. 15, the algorithm is locked into "*STATE_U*" since there is no negative event detected yet as demonstrated by the line representing the active state 1522 between samples 200-235. As shown in the first graph 1510 of Fig. 15, x[n] drops below the noise threshold 1512 around sample n=235, and as shown in the second graph 1520 of Fig. 15, there is a state transition from "*STATE_U*" to "*STATE_UD*" as demonstrated by the line representing the active state 1522 around sample n=235. This means that the duration of the positive pulse detected was sufficient so that it qualifies as an R-wave candidate. As shown in the first graph 1510 of Fig. 15, x[n] remains lower than the noise threshold 1512 between samples 235-302, and as shown in the second graph 1520 of Fig. 15, the algorithm is locked into "*STATE_UD*" since there is no positive event detected yet as demonstrated by the line representing the active state 1522 between samples 235-302. As shown in the first graph 1510 of Fig. 15, x[n] exceeds the noise threshold 1512 around sample n=302, and as shown in the second graph 1520 of Fig. 15, there is a state transition from "*STATE_UD*" to "*STATE_UDU*" as demonstrated by the line representing the active state 1522 around sample n=302. As shown in the first graph 1510 of Fig. 15, x[n] remains greater than the noise threshold 1512 between samples 302-310, and as shown in the second graph 1520 of Fig. 15, the algorithm is locked into "*STATE_UDU*" since there is no negative event detected yet as demonstrated by the line representing the active state 1522 between samples 302-310. As shown in the first graph 1510 of Fig. 15, x[n] drops below the noise threshold 1512 around sample n=310, and as shown in the second graph 1520 of Fig. 15, there is a state transition from "*STATE_UDU*" to "*STATE_UD*" as demonstrated by the line representing the active state 1522 around sample n=310. This means that the duration of the positive pulse detected was insufficient to qualify it as an R-wave candidate. After several intermediate transitions, as shown in the second graph 1520 of Fig. 15, the state "*STATE_UDUD*" is reached twice (at around samples 330 and 345). According to the algorithm, there is enough information to compute two RR-intervals, but they are flagged as low-quality and thus the background of the figure is shaded as described above.

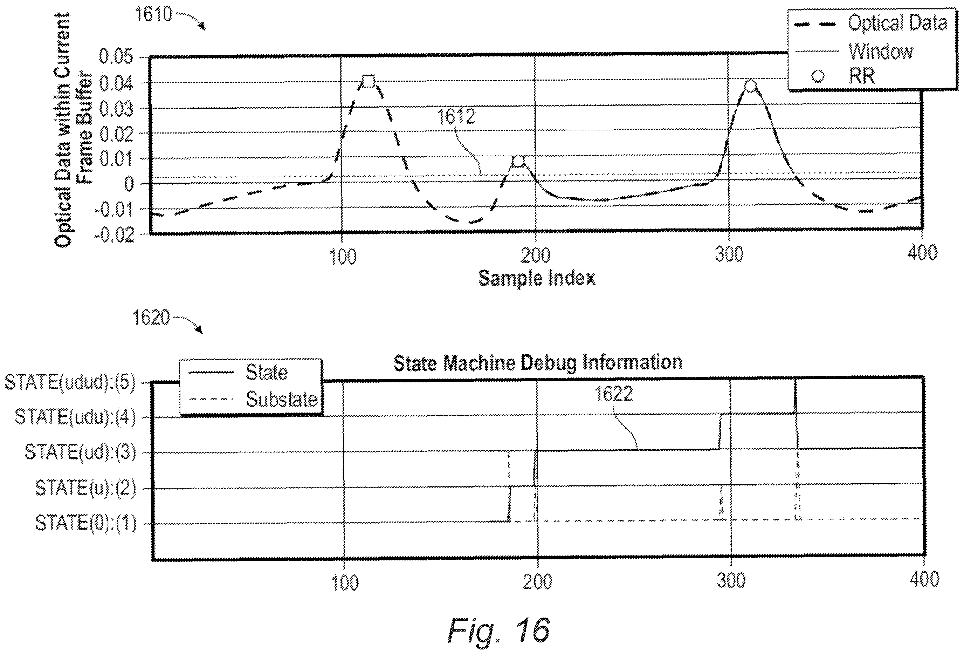

The third example corresponds to Fig. 16. Fig. 16 shows a first graph including a time domain buffer of optical data and a second graph showing state machine debugging information. Specifically, Fig. 16 represents a single packet of data corresponding to a time domain buffer of optical data as shown in the first graph 1610 and debug information associated with the state machine described herein as shown in the second graph 1620.

As shown in the second graph 1620 of Fig. 16, for the current packet, the state machine starts processing from sample $n = n_0 = 175$, and the first state is always "*STATE 0*". As shown in the first graph 1610 of Fig. 16, x[n] remains lower than the noise threshold 1612 between samples 175-186, and as shown in the second graph 1620 of Fig. 16, the algorithm is locked into "*STATE_0*" since there is no positive event detected yet as demonstrated by the line representing the active state 1622 between samples 175-186. As shown in the first graph 1610 of Fig. 16, x[n] exceeds the noise threshold 1612 around sample n=186, and as shown in the second graph 1620 of Fig. 16, there is a state transition from "*STATE_0*" to "*STATE_U*" as demonstrated by the line representing the active state 1622 around sample n=186. As shown in the first graph 1610 of Fig. 16, x[n] remains greater than the noise threshold 1612 between samples 186-200, and as shown in the second graph 1620 of Fig. 16, the algorithm is locked into "*STATE_U*" since there is no negative event detected yet as demonstrated by the line representing the active state 1622 between samples 186-200. As shown in the first graph 1610 of Fig. 16, x[n] drops below the noise threshold 1612 around sample n=200, and as shown in the second graph 1620 of Fig. 16, there is a state transition from "*STATE_U*" to "*STATE_UD*" as demonstrated by the line representing the active state 1622 around sample n=200. This means that the duration of the positive pulse detected was sufficient so that it qualifies as an R-wave candidate. As shown in the first graph 1610 of Fig. 16, x[n] remains lower than the noise threshold 1612 between samples 200-295, and as shown in the second graph 1620 of Fig. 16, the algorithm is locked into "*STATE_UD*" since there is no positive event detected yet as demonstrated by the line representing the active state 1622 between samples 200-295. As shown in the first graph 1610 of Fig. 16, x[n] exceeds the noise threshold 1612 around sample n=295, and as shown in the second graph 1620 of Fig. 16, there is a state transition from "*STATE_UD*" to "*STATE_UDU*" as demonstrated by the line representing the active state 1622 around sample n=295. As shown in the first graph 1610 of Fig. 16, x[n] remains greater than the noise threshold 1612 between samples 295-335, and as shown in the second graph 1620 of Fig. 16, the algorithm is locked into "*STATE_UDU*" since there is no negative event detected yet as demonstrated by the line representing the active state 1622 between samples 295-335. As shown in the first graph 1610 of Fig. 16, x[n] drops below the noise threshold 1612 around sample n=335, and as shown in the second graph 1620 of Fig. 16, there is a state transition from "*STATE_UDU*" to "*STATE_UDUD*" as demonstrated by the line representing the active state 1622 around sample n=335. This means that the duration of the positive pulse detected was sufficient so that it qualifies as a second R-wave candidate. Thus, at around sample=335, two consecutive R-pulse candidates "*STATE_UDUD*" have been detected. The areas of the two pulses are similar so that the state machine transitions back to state "*STATE_UD*" as demonstrated by the line representing the active state 1622. This allows the computation of an RR-interval indicated by the peaks in the first graph 1610. The RR-interval quality, however, is low and thus the background of the first graph 1610 is shaded. As shown in the first graph 1610 of Fig. 16, x[n] remains lower than the noise threshold 1612 between samples 335-400, and as shown in the second graph 1620 of Fig. 16, the algorithm is locked into "*STAT_UD*" since there is no positive event detected yet as demonstrated by the line representing the active state 1622 between samples 335-400. Thus, the end of the time domain buffer is reached, and there was enough data to compute one RR-interval where its associated quality was low. The search will resume on the next packet starting from index: $n_0 = 184$.

It will be generally noted from the examples above that pulse wave peaks for heart rate signals of various shapes can be accurately captured using state machines such as those described above to process time domain data. This includes signals that might result in failed detections or that might require frequency domain techniques to extract peak-to-peak estimates, such as noisy signals or signals with uncharacteristic peaks such as small intermediate peaks, multiple false peaks, and so forth. As a significant advantage, this more generally facilitates peak detection and HRV measurements for a significantly wider range of user contexts resulting from, e.g., high-motion activities, unusual fitness levels, cardiac pathologies, and so forth.

The above systems, devices, methods, processes, and the like may be realized in hardware, software, or any combination of these suitable for a particular application. The hardware may include a general-purpose computer and/or dedicated computing device. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software. In another aspect, the methods may be embodied in systems that perform the steps thereof, and may be distributed across devices in a number of ways. At the same time, processing may be distributed across devices such as the various systems described above, or all of the functionality may be integrated into a dedicated, standalone device or other hardware. In another aspect, means for performing the steps associated with the processes described above may include any of the hardware and/or software described above. All such permutations and combinations are intended to fall within the scope of the present disclosure.

Thus, in one aspect, each method described above and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above. All such permutations and combinations are intended to fall within the scope of the present disclosure.

The method steps of the implementations described herein are intended to include any suitable method of causing such method steps to be performed, consistent with the patentability of the following claims, unless a different meaning is expressly provided or otherwise clear from the context. So for example performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity, and need not be located within a particular jurisdiction. It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the scope of this disclosure and are intended to form a part of the invention as defined by the following claims, which are to be interpreted in the broadest sense allowable by law.

**Claims**

1. A computer program product for operating a wearable, continuous physiological monitoring system, the computer program product comprising computer executable code that, when executing on the wearable, continuous physiological monitoring system, performs the steps of:

   storing a window of time domain data (1202) for a physiological signal, the window including a series of samples of the physiological signal;
   detecting a first peak and a second peak in the physiological signal (1204) using a time domain process on the series of samples, wherein the time domain process includes a first state machine (800) configured to sequentially process the series of samples and identify the first peak and the second peak in the series of samples;
   evaluating a quality of the time domain data in the window (1206) indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine (900);

locally refining a peak position of each of the first peak and the second peak (1208), wherein locally refining the peak position includes interpolating a location of a maximum for a number of sequential samples within the time domain data having similar magnitudes; and

when the quality exceeds a predetermined threshold, conditionally calculating a physiological interval for the window of time domain data (1210) wherein the physiological interval is based on a time between the first peak and the second peak and storing a value based on the quality as a quality flag for the window of time domain data.

2. The computer program product of claim 1, wherein the physiological signal is a heart rate signal, and wherein evaluating the quality of the time domain data further includes conditionally labelling the quality of the time domain data as low-RR-quality when the difference in amplitudes of the first local maximum and the second local maximum are within a predetermined threshold.

3. The computer program product of any of claims 1 and 2, wherein the first state machine (800) is further configured to perform the steps of:

identifying a first pulse candidate, wherein the first pulse candidate is a heart rate signal;

conditionally labelling the first pulse candidate as the first peak when the first pulse candidate is determined to be an R-pulse;

identifying a second pulse candidate, wherein the second pulse candidate is a heart rate signal; and

conditionally labelling the second pulse candidate as the second peak when the second pulse candidate is determined to be an R-pulse.

4. A method comprising:

storing a window of time domain data (1202) for a physiological signal, the window including a series of samples of the physiological signal;

detecting a first peak and a second peak in the physiological signal (1204) using a time domain process on the series of samples;

wherein the time domain process includes a first state machine (800) configured to sequentially process the series of samples and identify the first peak and the second peak in the series of samples;

evaluating a quality of the time domain data in the window (1206) indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine (900);

locally refining a peak position of each of the first peak and the second peak (1208), wherein locally refining the peak position includes interpolating a location of a maximum for a number of sequential samples within the time domain data having similar magnitudes; and

when the quality exceeds a predetermined threshold, conditionally calculating a physiological interval for the window of time domain data (1210) wherein the physiological interval is based on a time between the first peak and the second peak and storing a value based on the quality as a quality flag for the window of time domain data.

5. The method of claim 4, wherein the physiological signal is a heart rate signal, and wherein evaluating the quality of the time domain data further includes conditionally labelling the quality of the time domain data as low-RR-quality when the difference in amplitudes of the first local maximum and the second local maximum are within a predetermined threshold.

6. The method of claim 4 or 5, wherein locally refining the peak position includes estimating a value of a maximum at a location for the maximum between two sequential samples within the time domain data.

7. The method of claim 4, wherein the first state machine (800) is further configured to perform the steps of:

identifying a first pulse candidate, wherein the first pulse candidate is a heart rate signal;

conditionally labelling the first pulse candidate as the first peak when the first pulse candidate is determined to be an R-pulse;

identifying a second pulse candidate, wherein the second pulse candidate is a heart rate signal; and

conditionally labelling the second pulse candidate as the second peak when the second pulse candidate is determined to be an R-pulse.

8. The method of claim 7, wherein the first state machine (800) is further configured to compare second-order statistics of the first peak and the second peak.

9. A system comprising:

a wearable housing;
one or more sensors (202) in the wearable housing for capturing physiological data; and
a processor (208) in the wearable housing, the processor configured by computer executable code to perform the steps of:

storing a window of time domain data (1202) for a physiological signal, the window including a series of samples of the physiological signal;
detecting a first peak and a second peak in the physiological signal using a time domain process on the series of samples (1204), wherein the time domain process includes a first state machine (800) configured to sequentially process the series of samples and identify the first peak and the second peak in the series of samples;
evaluating a quality of the time domain data in the window (1206) indicative of how well a time domain shape associated with a peak shape matches an expected peak shape for the physiological signal, wherein evaluating a quality of the time domain data includes identifying the peak shape by identifying a local minimum located between a first local maximum and a second local maximum with a second state machine (900);
locally refining a peak position of each of the first peak and the second peak (1208), wherein locally refining the peak position includes interpolating a location of a maximum for a number of sequential samples within the time domain data having similar magnitudes; and
when the quality exceeds a predetermined threshold, conditionally calculating a physiological interval for the window of time domain data (1210) wherein the physiological interval is based on a time between the first peak and the second peak and storing a value based on the quality as a quality flag for the window of time domain data.

10. The system of claim 9, wherein locally refining the peak position includes estimating a value of a maximum at a location for the maximum between two sequential samples of the time domain data.

11. The system of claim 9, wherein the first state machine (800) is further configured to perform the steps of:

identifying a first pulse candidate, wherein the first pulse candidate is a heart rate signal;
conditionally labelling the first pulse candidate as the first peak when the first pulse candidate is determined to be an R-pulse;
identifying a second pulse candidate, wherein the second pulse candidate is a heart rate signal; and
conditionally labelling the second pulse candidate as the second peak when the second pulse candidate is determined to be an R-pulse.


**Patentansprüche**

1. Computerprogrammprodukt zum Betreiben eines tragbaren, kontinuierlichen physiologischen Überwachungssystems, wobei das Computerprogrammprodukt einen computerausführbaren Code umfasst, der, wenn er auf dem tragbaren, kontinuierlichen physiologischen Überwachungssystem ausgeführt wird, die folgenden Schritte durchführt:

Speichern eines Fensters von Zeitbereichsdaten (1202) für ein physiologisches Signal, wobei das Fenster eine Reihe von Abtastwerten des physiologischen Signals enthält;
Erfassen einer ersten Spitze und einer zweiten Spitze in dem physiologischen Signal (1204) unter Verwendung eines Zeitbereichsprozesses an der Reihe von Abtastwerten, wobei der Zeitbereichsprozess eine erste Zustandsmaschine (800) umfasst, die so konfiguriert ist, dass sie die Reihe von Abtastwerten sequentiell verarbeitet und die erste Spitze und die zweite Spitze in der Reihe von Abtastwerten identifiziert;
Auswerten einer Qualität der Zeitbereichsdaten in dem Fenster (1206), die anzeigt, wie gut eine Zeitbereichsform, die mit einer Spitzenform verbunden ist, mit einer erwarteten Spitzenform für das physiologische Signal übereinstimmt, wobei das Auswerten einer Qualität der Zeitbereichsdaten das Identifizieren der Spitzenform durch Identifizieren eines lokalen Minimums, das zwischen einem ersten lokalen Maximum und einem zweiten lokalen Maximum liegt, mit einer zweiten Zustandsmaschine (900) umfasst;

lokales Verfeinern einer Spitzenposition sowohl der ersten Spitze als auch der zweiten Spitze (1208), wobei das lokale Verfeinern der Spitzenposition das Interpolieren einer Position eines Maximums für eine Anzahl von aufeinanderfolgenden Abtastwerten innerhalb der Zeitbereichsdaten mit ähnlichen Größen beinhaltet; und wenn die Qualität einen vorbestimmten Schwellenwert überschreitet, bedingtes Berechnen eines physiologischen Intervalls für das Fenster von Zeitbereichsdaten (1210), wobei das physiologische Intervall auf einer Zeit zwischen der ersten Spitze und der zweiten Spitze basiert, und Speichern eines auf der Qualität basierenden Wertes als ein Qualitätsindikator für das Fenster von Zeitbereichsdaten.

2. Computerprogrammprodukt nach Anspruch 1, wobei das physiologische Signal ein Herzfrequenzsignal ist, und wobei das Auswerten der Qualität der Zeitdomänendaten ferner das bedingte Kennzeichnen der Qualität der Zeitdomänendaten als Niedrig-RR-Qualität umfasst, wenn die Differenz der Amplituden des ersten lokalen Maximums und des zweiten lokalen Maximums innerhalb eines vorbestimmten Schwellenwerts liegt.

3. Computerprogrammprodukt nach einem der Ansprüche 1 und 2, wobei die erste Zustandsmaschine (800) ferner so konfiguriert ist, dass sie die folgenden Schritte ausführt:

Identifizieren eines ersten Pulskandidaten, wobei der erste Pulskandidat ein Herzfrequenzsignal ist;
bedingtes Kennzeichnen des ersten Pulskandidaten als die erste Spitze, wenn der erste Pulskandidat als ein R-Puls bestimmt wird;
Identifizieren eines zweiten Pulskandidaten, wobei der zweite Pulskandidat ein Herzfrequenzsignal ist; und
bedingtes Kennzeichnen des zweiten Pulskandidaten als die zweite Spitze, wenn der zweite Pulskandidat als R-Puls bestimmt wird.

4. Ein Verfahren, das Folgendes umfasst:

Speichern eines Fensters von Zeitbereichsdaten (1202) für ein physiologisches Signal, wobei das Fenster eine Reihe von Abtastwerten des physiologischen Signals enthält;
Erfassen einer ersten Spitze und einer zweiten Spitze in dem physiologischen Signal (1204) unter Verwendung eines Zeitbereichsprozesses an der Reihe von Abtastwerten;
wobei der Zeitbereichsprozess eine erste Zustandsmaschine (800) enthält, die so konfiguriert ist, dass sie die Reihe von Abtastwerten sequentiell verarbeitet und den ersten Spitzenwert und den zweiten Spitzenwert in der Reihe von Abtastwerten identifiziert;
Auswerten einer Qualität der Zeitbereichsdaten in dem Fenster (1206), die anzeigt, wie gut eine Zeitbereichsform, die mit einer Spitzenform verbunden ist, mit einer erwarteten Spitzenform für das physiologische Signal übereinstimmt, wobei das Auswerten einer Qualität der Zeitbereichsdaten das Identifizieren der Spitzenform durch Identifizieren eines lokalen Minimums, das zwischen einem ersten lokalen Maximum und einem zweiten lokalen Maximum liegt, mit einer zweiten Zustandsmaschine (900) umfasst;
lokales Verfeinern einer Spitzenposition sowohl der ersten Spitze als auch der zweiten Spitze (1208), wobei das lokale Verfeinern der Spitzenposition das Interpolieren einer Position eines Maximums für eine Anzahl von aufeinanderfolgenden Abtastwerten innerhalb der Zeitbereichsdaten mit ähnlichen Größen beinhaltet; und wenn die Qualität einen vorbestimmten Schwellenwert überschreitet, bedingtes Berechnen eines physiologischen Intervalls für das Fenster von Zeitbereichsdaten (1210), wobei das physiologische Intervall auf einer Zeit zwischen der ersten Spitze und der zweiten Spitze basiert, und Speichern eines auf der Qualität basierenden Wertes als ein Qualitätsindikator für das Fenster von Zeitbereichsdaten.

5. Verfahren nach Anspruch 4, wobei das physiologische Signal ein Herzfrequenzsignal ist und wobei das Auswerten der Qualität der Zeitdomänendaten ferner das bedingte Kennzeichnen der Qualität der Zeitdomänendaten als Niedrig-RR-Qualität umfasst, wenn die Differenz der Amplituden des ersten lokalen Maximums und des zweiten lokalen Maximums innerhalb eines vorbestimmten Schwellenwertes liegt.

6. Verfahren nach Anspruch 4 oder 5, wobei das lokale Verfeinern der Spitzenposition das Schätzen eines Wertes eines Maximums an einer Stelle für das Maximum zwischen zwei aufeinanderfolgenden Abtastwerten innerhalb der Zeitbereichsdaten umfasst.

**7.** Verfahren nach Anspruch 4, wobei die erste Zustandsmaschine (800) ferner so konfiguriert ist, dass sie die folgenden Schritte ausführt

Identifizieren eines ersten Pulskandidaten, wobei der erste Pulskandidat ein Herzfrequenzsignal ist;
bedingtes Kennzeichnen des ersten Pulskandidaten als erste Spitze, wenn der erste Pulskandidat als R-Puls bestimmt wird;
Identifizieren eines zweiten Pulskandidaten, wobei der zweite Pulskandidat ein Herzfrequenzsignal ist; und
bedingtes Kennzeichnen des zweiten Pulskandidaten als die zweite Spitze, wenn der zweite Pulskandidat als R-Puls bestimmt wird.

**8.** Verfahren nach Anspruch 7, wobei die erste Zustandsmaschine (800) ferner so konfiguriert ist, dass sie Statistiken zweiter Ordnung der ersten Spitze und der zweiten Spitze vergleicht.

**9.** Ein System, das Folgendes umfasst:

ein tragbares Gehäuse;
einen oder mehrere Sensoren (202) in dem tragbaren Gehäuse zum Erfassen physiologischer Daten; und
einen Prozessor (208) in dem tragbaren Gehäuse, wobei der Prozessor durch einen computerausführbaren Code konfiguriert ist, um die folgenden Schritte auszuführen
Speichern eines Fensters von Zeitbereichsdaten (1202) für ein physiologisches Signal, wobei das Fenster eine Reihe von Abtastwerten des physiologischen Signals enthält;
Erfassen einer ersten Spitze und einer zweiten Spitze in dem physiologischen Signal unter Verwendung eines Zeitbereichsprozesses an der Reihe von Abtastwerten (1204), wobei der Zeitbereichsprozess eine erste Zustandsmaschine (800) enthält, die so konfiguriert ist, dass sie die Reihe von Abtastwerten sequentiell verarbeitet und der ersten Spitze und der zweiten Spitze in der Reihe von Abtastwerten identifiziert;
Auswerten einer Qualität der Zeitbereichsdaten in dem Fenster (1206), die anzeigt, wie gut eine Zeitbereichsform, die mit einer Spitzenform verbunden ist, mit einer erwarteten Spitzenform für das physiologische Signal übereinstimmt, wobei das Auswerten einer Qualität der Zeitbereichsdaten das Identifizieren der Spitzenform durch Identifizieren eines lokalen Minimums, das zwischen einem ersten lokalen Maximum und einem zweiten lokalen Maximum liegt, mit einer zweiten Zustandsmaschine (900) umfasst;
lokales Verfeinern einer Spitzenposition sowohl der ersten Spitze als auch der zweiten Spitze (1208), wobei das lokale Verfeinern der Spitzenposition das Interpolieren einer Position eines Maximums für eine Anzahl von aufeinanderfolgenden Abtastwerten innerhalb der Zeitbereichsdaten mit ähnlichen Größen beinhaltet; und
wenn die Qualität einen vorbestimmten Schwellenwert überschreitet, bedingtes Berechnen eines physiologischen Intervalls für das Fenster von Zeitbereichsdaten (1210), wobei das physiologische Intervall auf einer Zeit zwischen der ersten Spitze und der zweiten Spitze basiert, und Speichern eines auf der Qualität basierenden Wertes als ein Qualitätsindikator für das Fenster von Zeitbereichsdaten.

**10.** System nach Anspruch 9, wobei das lokale Verfeinern der Spitzenposition das Schätzen eines Wertes eines Maximums an einer Stelle für das Maximum zwischen zwei aufeinanderfolgenden Abtastwerten der Zeitbereichsdaten umfasst.

**11.** System nach Anspruch 9, wobei die erste Zustandsmaschine (800) ferner so konfiguriert ist, dass sie die folgenden Schritte ausführt

Identifizieren eines ersten Pulskandidaten, wobei der erste Pulskandidat ein Herzfrequenzsignal ist;
bedingtes Kennzeichnen des ersten Pulskandidaten als die erste Spitze, wenn der erste Pulskandidat als R-Puls bestimmt wird;
Identifizieren eines zweiten Pulskandidaten, wobei der zweite Pulskandidat ein Herzfrequenzsignal ist; und
bedingtes Kennzeichnen des zweiten Pulskandidaten als die zweite Spitze, wenn der zweite Pulskandidat als ein R-Puls bestimmt wird.

**Revendications**

**1.** Produit programme d'ordinateur pour l'exploitation d'un système de surveillance physiologique continue portable, le produit programme d'ordinateur comprenant un code exécutable par ordinateur qui, lors de son exécution sur le système de surveillance physiologique continue portable, réalise les étapes de :

stockage d'une fenêtre de données de domaine temporel (1202) pour un signal physiologique, la fenêtre comportant une série d'échantillons du signal physiologique ;

détection d'un premier pic et d'un second pic dans le signal physiologique (1204) en utilisant un traitement de domaine temporel sur la série d'échantillons, dans lequel le traitement de domaine temporel comporte une première machine à états (800) configurée pour traiter séquentiellement la série d'échantillons et identifier le premier pic et le second pic dans la série d'échantillons ;

évaluation d'une qualité des données de domaine temporel dans la fenêtre (1206) indicative d'à quel point une forme de domaine temporel associée à une forme de pic correspond bien à une forme de pic attendue pour le signal physiologique, dans lequel l'évaluation d'une qualité des données de domaine temporel comporte l'identification de la forme de pic par l'identification d'un minimum local situé entre un premier maximum local et un second maximum local avec une seconde machine à états (900) ;

affinement local d'une position de pic de chacun du premier pic et du second pic (1208), dans lequel l'affinement local de la position de pic comporte l'interpolation d'un emplacement d'un maximum pour un nombre d'échantillons séquentiels au sein des données de domaine temporel ayant des amplitudes similaires ; et

lorsque la qualité dépasse un seuil prédéterminé, calcul de manière conditionnelle d'un intervalle physiologique pour la fenêtre de données de domaine temporel (1210) dans lequel l'intervalle physiologique est basé sur un temps entre le premier pic et le second pic et le stockage d'une valeur basée sur la qualité comme étant un drapeau de qualité pour la fenêtre de données de domaine temporel.

2. Produit programme d'ordinateur selon la revendication 1, dans lequel le signal physiologique est un signal de fréquence cardiaque, et dans lequel l'évaluation de la qualité des données de domaine temporel comporte en outre l'étiquetage de manière conditionnelle de la qualité des données de domaine temporel comme étant de faible qualité de RR lorsque les différences dans les amplitudes du premier maximum local et du second maximum local sont en deçà d'un seuil prédéterminé.

3. Produit programme d'ordinateur selon l'une des revendications 1 et 2, dans lequel la première machine à états (800) est en outre configurée pour réaliser les étapes de :

identification d'un premier candidat de pouls, dans lequel le premier candidat de pouls est un signal de fréquence cardiaque ;

étiquetage de manière conditionnelle du premier candidat de pouls comme étant le premier pic lorsque le premier candidat de pouls est déterminé comme étant un pouls R ;

identification d'un second candidat de pouls, dans lequel le second candidat de pouls est un signal de fréquence cardiaque ; et

étiquetage de manière conditionnelle du second candidat de pouls comme étant le second pic lorsque le second candidat de pouls est déterminé comme étant un pouls R.

4. Procédé comprenant :

le stockage d'une fenêtre de données de domaine temporel (1202) pour un signal physiologique, la fenêtre comportant une série d'échantillons du signal physiologique ;

la détection d'un premier pic et d'un second pic dans le signal physiologique (1204) en utilisant un traitement de domaine temporel sur la série d'échantillons ;

dans lequel le traitement de domaine temporel comporte une première machine à états (800) configurée pour traiter séquentiellement la série d'échantillons et identifier le premier pic et le second pic dans la série d'échantillons ;

l'évaluation d'une qualité des données de domaine temporel dans la fenêtre (1206) indicative d'à quel point une forme de domaine temporel associée à une forme de pic correspond bien à une forme de pic attendue pour le signal physiologique, dans lequel l'évaluation d'une qualité des données de domaine temporel comporte l'identification de la forme de pic par l'identification d'un minimum local situé entre un premier maximum local et un second maximum local avec une seconde machine à états (900) ;

l'affinement local d'une position de pic de chacun du premier pic et du second pic (1208), dans lequel l'affinement local de la position de pic comporte l'interpolation d'un emplacement d'un maximum pour un nombre d'échantillons séquentiels au sein des données de domaine temporel ayant des amplitudes similaires ; et

lorsque la qualité dépasse un seuil prédéterminé, le calcul de manière conditionnelle d'un intervalle physiologique pour la fenêtre de données de domaine temporel (1210) dans lequel l'intervalle physiologique est basé sur un temps entre le premier pic et le second pic et le stockage d'une valeur basée sur la qualité comme étant un drapeau de qualité pour la fenêtre de données de domaine temporel.

**5.** Procédé selon la revendication 4, dans lequel le signal physiologique est un signal de fréquence cardiaque, et dans lequel l'évaluation de la qualité des données de domaine temporel comporte en outre l'étiquetage de manière conditionnelle de la qualité des données de domaine temporel comme étant de faible qualité de RR lorsque les différences dans les amplitudes du premier maximum local et du second maximum local sont en deçà d'un seuil prédéterminé.

**6.** Procédé selon la revendication 4 ou 5, dans lequel l'affinement local de la position de pic comporte l'estimation d'une valeur d'un maximum à un emplacement pour le maximum entre deux échantillons séquentiels au sein des données de domaine temporel.

**7.** Procédé selon la revendication 4, dans lequel la première machine à états (800) est en outre configurée pour réaliser les étapes de :

identification d'un premier candidat de pouls, dans lequel le premier candidat de pouls est un signal de fréquence cardiaque ;
étiquetage de manière conditionnelle du premier candidat de pouls comme étant le premier pic lorsque le premier candidat de pouls est déterminé comme étant un pouls R ;
identification d'un second candidat de pouls, dans lequel le second candidat de pouls est un signal de fréquence cardiaque ; et
étiquetage de manière conditionnelle du second candidat de pouls comme étant le second pic lorsque le second candidat de pouls est déterminé comme étant un pouls R.

**8.** Procédé selon la revendication 7, dans lequel la première machine à états (800) est en outre configurée pour comparer des statistiques de second ordre du premier pic et du second pic.

**9.** Système comprenant :

un boîtier portable ;
un ou plusieurs capteurs (202) dans le boîtier portable pour la capture de données physiologiques ; et
un processeur (208) dans le boîtier portable, le processeur étant configuré par un code exécutable par ordinateur pour réaliser les étapes de :

stockage d'une fenêtre de données de domaine temporel (1202) pour un signal physiologique, la fenêtre comportant une série d'échantillons du signal physiologique ;
détection d'un premier pic et d'un second pic dans le signal physiologique en utilisant un traitement de domaine temporel sur la série d'échantillons (1204), dans lequel le traitement de domaine temporel comporte une première machine à états (800) configurée pour traiter séquentiellement la série d'échantillons et identifier le premier pic et le second pic dans la série d'échantillons ;
évaluation d'une qualité des données de domaine temporel dans la fenêtre (1206) indicative d'à quel point une forme de domaine temporel associée à une forme de pic correspond bien à une forme de pic attendue pour le signal physiologique, dans lequel l'évaluation d'une qualité des données de domaine temporel comporte l'identification de la forme de pic par l'identification d'un minimum local situé entre un premier maximum local et un second maximum local avec une seconde machine à états (900) ;
affinement local d'une position de pic de chacun du premier pic et du second pic (1208), dans lequel l'affinement local de la position de pic comporte l'interpolation d'un emplacement d'un maximum pour un nombre d'échantillons séquentiels au sein des données de domaine temporel ayant des amplitudes similaires ; et
lorsque la qualité dépasse un seuil prédéterminé, calcul de manière conditionnelle d'un intervalle physiologique pour la fenêtre de données de domaine temporel (1210) dans lequel l'intervalle physiologique est basé sur un temps entre le premier pic et le second pic et le stockage d'une valeur basée sur la qualité comme étant un drapeau de qualité pour la fenêtre de données de domaine temporel.

**10.** Système selon la revendication 9, dans lequel l'affinement local de la position de pic comporte l'estimation d'une valeur d'un maximum à un emplacement pour le maximum entre deux échantillons séquentiels des données de domaine temporel.

**11.** Système selon la revendication 9, dans lequel la première machine à états (800) est en outre configurée pour réaliser les étapes suivantes :

identification d'un premier candidat de pouls, dans lequel le premier candidat de pouls est un signal de fréquence cardiaque ;

étiquetage de manière conditionnelle du premier candidat de pouls comme étant le premier pic lorsque le premier candidat de pouls est déterminé comme étant un pouls R ;

identification d'un second candidat de pouls, dans lequel le second candidat de pouls est un signal de fréquence cardiaque ; et

étiquetage de manière conditionnelle du second candidat de pouls comme étant le second pic lorsque le second candidat de pouls est déterminé comme étant un pouls R.

*Fig. 1*

EP 4 243 694 B1

*Fig. 2*

EP 4 243 694 B1

*Fig. 3*

CONVERT HEART RATE READINGS INTO HEART RATE RESERVE VALUES
402

WEIGHT HEART RATE RESERVE VALUES ACCORDING TO WEIGHTING SCHEME
404

SUM AND NORMALIZE WEIGHTED TIME SERIES OF HEART RATE RESERVE VALUES
406

SCALE SUMMED AND NORMALIZED VALUES TO GENERATE INTENSITY SCORE
408

STORE INTENSITY SCORE ON NON-TRANSITORY COMPUTER-READABLE STORAGE MEDIUM
410

DISPLAY INTENSITY SCORE ON USER INTERFACE RENDERED ON DISPLAY DEVICE
412

*Fig. 4*

EP 4 243 694 B1

```
                                                                        ⌇ 500
┌─────────────────────────────────────────────────────────────┐
│  DETERMINE HEART RATE VARIABILITY BASED ON CONTINUOUS        │
│                    HEART RATE DATA                            │
│                        502                                   │
└─────────────────────────────────────────────────────────────┘
                              │
                              ▼
┌─────────────────────────────────────────────────────────────┐
│           GENERATE AND DISPLAY INTENSITY SCORE               │
│                        504                                   │
└─────────────────────────────────────────────────────────────┘
                              │
                              ▼
┌─────────────────────────────────────────────────────────────┐
│  GENERATE AND ADJUST AN EXERCISE ROUTINE BASED ON            │
│                    INTENSITY SCORE                           │
│                        506                                   │
└─────────────────────────────────────────────────────────────┘
                              │
                              ▼
┌─────────────────────────────────────────────────────────────┐
│           GENERATE AND DISPLAY A RECOVERY SCORE              │
│                        508                                   │
└─────────────────────────────────────────────────────────────┘
                              │
                              ▼
         YES    ◇ RECOVERY SCORE > FIRST THRESHOLD? ◇    NO
        ┌───────────         510           ─────────────┐
        ▼                                                │
┌──────────────────────────────┐                        │
│ INDICATE THAT USER IS         │                        │
│ EXERCISE-READY                │                        │
│        512                    │                        ▼
└──────────────────────────────┘    NO  ◇ RECOVERY SCORE < SECOND THRESHOLD? ◇  YES
                              ┌─────────────       514        ─────────────┐
                              ▼                                            ▼
            ┌──────────────────────────────┐    ┌──────────────────────────────┐
            │ INDICATE THAT USER MAY        │    │ INDICATE THAT USER IS NOT     │
            │ EXERCISE WITH CARE            │    │ EXERCISE-READY                │
            │        518                    │    │        516                    │
            └──────────────────────────────┘    └──────────────────────────────┘
```

*Fig. 5*

600

DETECT SLEEP STATE OF USER
602

↓

MONITOR HEART RATE OF USER
604

↓

RECORD HEART RATE AS HEART RATE
DATA
606

↓

DETECT WAKING EVENT
610

CALCULATE HEART RATE VARIABILITY
IN LAST PHASE OF SLEEP
612

↓

CALCULATE DURATION OF SLEEP
STATE
614

↓

EVALUATE QUALITY OF HEART RATE
DATA USING DATA QUALITY METRIC
618

↓

CALCULATE RECOVERY SCORE(S)
620

↓

CALCULATE SLEEP SCORES
630

*Fig. 6*

EP 4 243 694 B1

*Fig. 7*

Fig. 8

Fig. 9

Fig. 10

Fig. 11

```
                    STORE WINDOW OF TIME DOMAIN DATA          1200
                       FOR PHYSIOLOGICAL SIGNAL
                                 1202

                    DETECT FIRST AND SECOND PEAKS IN
                          PHYSIOLOGICAL SIGNAL
                                 1204

                    EVALUATE QUALITY OF TIME DOMAIN
                                 DATA
                                 1206

                         REFINE PEAK POSITIONS
                                 1208

                      CONDITIONALLY CALCULATE
                   PHYSIOLOGICAL INTERVAL AND STORE
                                QUALITY
                                 1210
```

*Fig. 12*

Fig. 13

Fig. 14

Fig. 15

EP 4 243 694 B1

Fig. 16

## REFERENCES CITED IN THE DESCRIPTION

### Patent documents cited in the description

- US 63121636

### Non-patent literature cited in the description

- An automated method for detecting systolic peaks from arterial blood pressure signals. **RAJU DANDU SRIRAM et al.** PROCEEDINGS OF THE 2014 IEEE STUDENTS' TECHNOLOGY SYMPOSIUM. IEEE, 28 February 2014

- **A. GRABOVSKIS et al.** Photoplethysmography system for blood pulsation detection in unloaded artery conditions. *SPIE PROCEEDINGS,* 26 April 2012, vol. 8427